(19) Europäisches Patentamt
European Patent Office
Office européen des brevets

(11) EP 3 558 219 B1

(12) EUROPEAN PATENT SPECIFICATION

(45) Date of publication and mention of the grant of the patent:
**15.10.2025 Bulletin 2025/42**

(21) Application number: **17818555.9**

(22) Date of filing: **19.12.2017**

(51) International Patent Classification (IPC):
***A61K 8/24*** (2006.01) ***A61Q 5/08*** (2006.01)
***A61K 8/19*** (2006.01) ***A61K 8/22*** (2006.01)

(52) Cooperative Patent Classification (CPC):
**A61K 8/24; A61K 8/19; A61K 8/22; A61Q 5/08**

(86) International application number:
**PCT/EP2017/083421**

(87) International publication number:
**WO 2018/114873 (28.06.2018 Gazette 2018/26)**

(54) **HAIR LIGHTENING COMPOSITION COMPRISING AN OXIDIZING AGENT, A CARBONATE AND AT LEAST 0.5% BY WEIGHT OF POLYPHOSPHORUS DERIVATIVE**

HAARAUFHELLUNGSZUSAMMENSETZUNG MIT EINEM OXIDATIONSMITTEL, EINEM CARBONAT UND MINDESTENS 0.5 % POLYPHOSPHORDERIVATGEWICHT

COMPOSITION D’ÉCLAIRCISSEMENT DES CHEVEUX COMPRENANT UN AGENT OXYDANT, UN CARBONATE ET AU MOINS 0.5 % EN POIDS D’UN DÉRIVÉ POLYPHOSPHORÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2016 FR 1662844**

(43) Date of publication of application:
**30.10.2019 Bulletin 2019/44**

(73) Proprietor: **L’OREAL**
**75008 Paris (FR)**

(72) Inventors:
- **LAGRANGE, Alain**
  **93400 Saint-Ouen (FR)**
- **LALLEMAN, Boris**
  **93400 Saint-Ouen (FR)**

(74) Representative: **L’Oreal**
**Service D.I.P.I.**
**9, rue Pierre Dreyfus**
**92110 Clichy (FR)**

(56) References cited:
**WO-A1-2014/207097** **FR-A- 1 070 766**
**FR-A1- 2 132 213** **FR-A1- 2 961 397**
**GB-A- 2 217 735** **US-A- 3 997 659**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to a composition for lightening keratin materials, preferably keratin fibres, in particular human keratin fibres such as the hair, comprising (a) hydrogen peroxide; (b) one or more carbonates and/or one or more carbonate-generating systems, (c) one or more polyphosphorus derivatives, in a total content of greater than or equal to 0.5% by weight, relative to the total weight of the composition, the pH of the composition being less than or equal to 9.7, preferably less than or equal to 9.5, and the composition being free of persalts.

The invention also relates to a lightening process using said composition, and also to a multi-compartment device which is suitable for using said lightening composition.

The present invention relates to the field of the lightening of keratin fibres and more particularly to the field of lightening the hair.

The lightening of hair is evaluated by the "tone depth", which characterizes the degree or level of lightening. The notion of "tone" is based on the classification of natural shades, one tone separating each shade from the shade immediately following or preceding it. This definition and the classification of natural shades are well known to hairstyling professionals and are published in the publication *Sciences des traitements capillaires* [Hair treatment science] by Charles Zviak, 1988, published by Masson, pages 215 and 278.

Tone depths range from 1 (black) to 10 (very light blond), one unit corresponding to one tone; the higher the figure, the lighter the shade.

Lightening makes it possible not only to afford a lighter tone depth than the initial natural tone depth of the head of hair, which is particularly sought by consumers, but also to afford visibility to a synthetic or natural colouring when this system is combined with dyes.

The processes for lightening human keratin fibres that are usually employed consist in using an aqueous composition comprising at least one oxidizing agent, under alkaline pH conditions in the vast majority of cases. This oxidizing agent has the role of degrading the melanin of the hair, which, depending on the nature of the oxidizing agent present, leads to more or less pronounced lightening of the fibres. Thus, for relatively mild lightening, the oxidizing agent is generally hydrogen peroxide. When more pronounced lightening is desired, use is usually made of peroxygenated salts, for instance persulfates, in the presence of hydrogen peroxide.

One of the difficulties arises from the fact that the lightening process is generally performed under highly alkaline conditions and that the alkaline agent most commonly used is aqueous ammonia. The use of aqueous ammonia is particularly advantageous in processes of this type. Specifically, it makes it possible to adjust the pH of the composition to an alkaline pH to enable activation of the oxidizing agent. However, this alkaline agent also causes swelling of the keratin fibre, with opening of the scales, which promotes the penetration of the oxidizing agent into the fibre, and thus increases the efficacy of the reaction.

However, this basifying agent is highly volatile, and this causes unpleasantness to the user on account of the strong and fairly unpleasant characteristic odour of ammonia that is given off during the process.

Furthermore, the amount of ammonia given off requires the use of higher contents than necessary in order to compensate for this loss. This is not without consequences for the user, who not only remains inconvenienced by the odour, but may also be confronted with greater risks of intolerance, for instance irritation of the scalp, which is reflected especially by stinging, and which also has an impact on the integrity of the fibre.

It has also been proposed to replace all or some of the aqueous ammonia with one or more other standard basifying agents, such as alkanolamines, but the solutions proposed hitherto do not result in compositions that are as effective as those based on aqueous ammonia, especially since these basifying agents do not provide sufficient lightening of pigmented fibres in the presence of the oxidizing agent. Furthermore, monoethanolamine, when used at high concentrations, may cause irritation of the scalp.

Thus, the hair-lightening technique, which must make it possible to obtain sufficient lightening of the fibre, generally involves using either aqueous ammonia or monoethanolamine, or else a mixture of monoethanolamine and aqueous ammonia, as basifying agent as a mixture with aqueous hydrogen peroxide solution.

FR2961397 has proposed to use compositions comprising a high amount of fatty substance, a surfactant, ammonium carbonate or bicarbonate, an organic base and oxidizing agent to obtain at least the same level of lightening as the lightening obtained with composition comprising a high concentration of ammonia.

Thus, one of the objectives of the present invention is to propose compositions for lightening keratin materials, preferably human keratin fibres such as the hair, which do not have the drawbacks mentioned above, i.e. which are capable of producing very good lightening performance while at the same time having working qualities that are superior to those of the existing compositions, especially while having a less disagreeable odour during their application to the fibres or during their preparation, and which are well tolerated by the scalp and respect the nature of the hair (integrity and sensory nature of the fibre). In particular, it is sought to obtain good lightening performance without using persalts.

These aims and others are achieved by the present invention, one subject of which is thus a cosmetic composition for lightening keratin materials, preferably keratin fibres, in particular human keratin fibres such as the hair,

comprising:

(a) hydrogen peroxide,
(b) one or more carbonates and/or one or more carbonate-generating systems,
(c) one or more polyphosphorus derivatives preferably chosen from linear or cyclic compounds comprising at least two phosphorus atoms covalently bonded together via at least one linker L comprising at least one oxygen atom and/or at least one carbon atom, preferably at least one oxygen atom, said polyphosphorus derivative(s) being present in a total content of greater than or equal to 0.5% by weight, relative to the total weight of the composition,

the pH of the composition being less than or equal to 9.7, preferably less than or equal to 9.5;
the composition being free of persalts.

For the purposes of the present invention, the term "composition for lightening" or "lightening composition" means a ready-to-use composition which is applied to keratin materials, preferably to keratin fibres and in particular to the hair. The lightening composition may be prepared just before application to said materials, preferably said keratin fibres.
A subject of the present invention is also a process for lightening keratin materials, preferably keratin fibres, in particular human keratin fibres such as the hair.
The invention also relates to a multi-compartment device for using the composition according to the invention.
The compositions according to the invention thus make it possible to produce very good lightening performance on keratin fibres, especially in terms of the level of lightening and the homogeneity of lightening, and also in terms of neutralization of glints (especially as regards the colour parameter b* in the CIE L*a*b* colour evaluation system) while at the same time preserving the integrity of the fibre (limiting the amount of breakage of the fibres) and conserving good sensory qualities of the fibre.
Moreover, the lightening process according to the invention also allows the use of compositions that do not have the drawbacks associated with odour during their application to keratin fibres or during their preparation.
Other characteristics and advantages of the invention will emerge more clearly on reading the description and the examples that follow.
In the text hereinbelow, and unless otherwise indicated, the limits of a range of values are included within that range.
The human keratin fibres treated via the process according to the invention are preferably the hair.
The expression *"at least one"* is equivalent to the expression *"one or more".*

***(a) chemical oxidizing agent***

The lightening composition according to the invention contains hydrogen peroxide as chemical oxidizing agent.
The term "chemical oxidizing agent" means an oxidizing agent other than atmospheric oxygen.
According to one particular embodiment, hydrogen peroxide may be added to the composition according to the invention just before its application to the keratin fibres. The intermediate composition(s) comprising hydrogen peroxide may be referred to as oxidizing compositions and may also contain various additional compounds or various adjuvants conventionally used in hair lightening compositions and as defined below.
According to a particular embodiment of the invention, hydrogen peroxide preferably represents a total hydrogen peroxide content of 0.1% to 25% by weight, preferably from 1% to 20% by weight or even more preferentially from 2% to 15% by weight, relative to the total weight of the lightening composition.
The composition according to the invention is free of peroxygenated salt(s) (also known as persalts).
According to a particularly preferred embodiment, the composition according to the invention is free of peroxygenated salt(s) or of peroxygenated salt generators, i.e. it comprises neither peroxygenated salts nor peroxygenated salt generators.

***Additional oxidizing agent:***

The lightening composition according to the invention may also comprise one or more additional oxidizing agents other than hydrogen peroxide and the peroxygenated salts mentioned previously.
More particularly, the additional oxidizing agent(s) are chosen from alkali metal bromates or ferricyanides.

***(b) carbonate(s) and/or carbonate-generating system***

The lightening system according to the present invention comprises one or more carbonates and/or one or more carbonate-generating systems.

The carbonate(s) or the carbonate-generating system(s) may be used in one or more cosmetic compositions during the lightening process.

The term "carbonate-generating system" means a system which generates carbonate in situ, for instance carbon dioxide in water.

The carbonate(s) are preferably chosen from:
a) carbonates of alkali metals ($Met_2^+$, $CO_3^{2-}$), of alkaline-earth metals ($Met'^{2+}$, $CO_3^{2-}$) or of phosphonium ($(R''_4P^+)_2$,$CO_3^{2-}$ with Met' representing an alkaline-earth metal and Met representing an alkali metal, and R'', which may be identical or different, representing a hydrogen atom or an optionally substituted ($C_1$-$C_6$)alkyl group such as hydroxyethyl; and mixtures thereof.

More particularly, the carbonate(s) are chosen from alkali metal and alkaline-earth metal carbonates; preferentially alkali metal carbonates, or mixtures thereof.

Preferably, they are chosen from carbonates ofNa, K, Mg, Ca and mixtures thereof.

Preferably, the carbonate(s) are chosen from sodium carbonate and potassium carbonate, and mixtures thereof.

According to the invention, the carbonate agent(s) used preferably represent a total content of from 0.01% to 20% by weight relative to the total weight of the composition according to the invention, and even more preferentially from 0.1% to 15% by weight.

Preferably, the carbonate agent(s) used represent a total content of from 0.5% to 10% by weight relative to the total weight of the composition according to the invention.

***<u>(c) polyphosphorus derivative(s)</u>***

In particular, the lightening composition according to the invention comprises c) one or more polyphosphorus derivatives in a total content of greater than or equal to 0.5% by weight relative to the total weight of the composition.

The term "polyphosphorus derivative" means preferably means linear or cyclic compounds comprising at least two phosphorus atoms bonded together covalently via at least one linker L comprising at least one oxygen atom and/or at least one carbon atom. According to one embodiment, when the linker comprises at least one carbon atom, it may comprise at least one nitrogen atom.

Particularly preferably, the linker L of the polyphosphorus derivatives used according to the various subjects of the invention comprises at least one oxygen atom.

Preferably, the polyphosphorus derivative(s) used according to the present invention comprise less than 20 phosphorus atoms, preferably less than 15 phosphorus atoms, preferably less than 10 phosphorus atoms.

Preferably, the polyphosphorus derivative comprises at least two groups chosen from a group -P(R)(=O)-OH, a group -P(R)(=O)-O M, a group >P(=O)-OH and/or a group >P(=O)-O M, with:

- M representing a cationic counterion, preferably chosen from alkali metals and alkaline-earth metals,
- R representing a hydroxyl group, a group $-O^-$ M, with M representing a cationic counterion, preferably chosen from alkali metals and alkaline-earth metals, a ($C_1$-$C_6$)alkyl, ($C_1$-$C_6$)alkoxy, cycloalkyloxy or (hetero)aryloxy group, and
- > representing the two bonds connected to the phosphorus atom and forming part of a ring.

According to the present invention, said polyphosphorus derivative(s) are present in a total content of greater than or equal to 0.5% by weight relative to the total weight of the composition.

According to a preferred embodiment, the polyphosphorus derivative(s) are chosen from inorganic polyphosphorus derivatives.

According to another embodiment, the polyphosphorus derivative(s) are chosen from organic polyphosphorus derivatives.

Preferably, the polyphosphorus derivative(s) present in the compositions according to the invention are non-amine derivatives.

Preferably, the polyphosphorus derivative(s) are chosen from polyphosphates and polyphosphonates, and mixtures thereof.

Preferably, the polyphosphorus derivative(s) are polyphosphates.

Preferably, the polyphosphorus derivative(s) c) as defined previously are chosen from:

- inorganic polyphosphorus derivatives chosen from:

    o pyrophosphates, preferably in the form of salts, preferably of alkali metal salts, which may or may not be hydrated, such as sodium pyrophosphate, potassium pyrophosphate or sodium pyrophosphate decahydrate;
    o hexametaphosphates, preferably in the form of salts, preferably of alkali metal salts, which may or may not be hydrated, such as sodium hexametaphosphate;

◦ tripolyphosphates, preferably in the form of salts, preferably of alkali metal salts, which may or may not be hydrated, such as sodium tripolyphosphate;
◦ trimetaphosphates, preferably in the form of salts, preferably of alkali metal salts, which may or may not be hydrated, such as sodium trimetaphosphate;
o and mixtures thereof;

- and organic polyphosphorus derivatives, preferably chosen from:

◦ organic polyphosphate derivatives, such as polyphosphoric acids and/or salts thereof such as phytic acid (also known as myo-inositol hexaphosphoric acid);
◦ organic polyphosphonate derivatives, such as polyphosphonic acids and/or salts thereof such as EDTMP, DETMP, ATMP, HEDP, DTPMP, and mixtures thereof;

- and mixtures thereof.

Preferably, the polyphosphorus derivative(s) are chosen from:

- inorganic polyphosphate derivatives chosen from hydrated or non-hydrated alkali metal pyrophosphates, such as sodium pyrophosphate, potassium pyrophosphate, sodium pyrophosphate decahydrate; and polyphosphates, such as sodium hexametaphosphate, sodium polyphosphate, sodium tripolyphosphate, sodium trimetaphosphate; and mixtures thereof;
- and organic polyphosphorus derivatives chosen from organic polyphosphate derivatives such as polyphosphoric acids and/or salts thereof such as phytic acid (also known as myo-inositol hexaphosphoric acid), organic polyphosphonate derivatives such as polyphosphonic acids and/or salts thereof such as EDTMP, DETMP, ATMP, HEDP, DTPMP, and mixtures thereof;
- and mixtures thereof.

Preferably, the polyphosphorus derivative(s) are chosen from inorganic polyphosphate derivatives, preferably from hydrated or non-hydrated alkali metal pyrophosphates, preferably from sodium pyrophosphate, potassium pyrophosphate and sodium pyrophosphate decahydrate.
Preferably, said polyphosphorus derivative(s) are chosen from compounds belonging to any one of formulae (I), (II) and (III) below; or mixtures thereof:

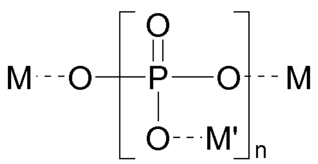
(I)

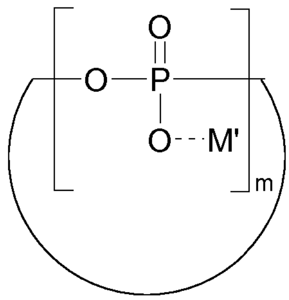
(II)

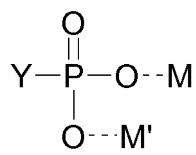
(III)

and also the solvates thereof such as hydrates;
with

- n ranging from 2 to 10, preferably from 2 to 6 and better still from 2 to 3;
- m ranging from 2 to 10, preferably from 2 to 6;
Y representing an alkyl chain comprising at least one phosphorus atom and optionally one or more non-phosphorus heteroatoms, or a cyclic carbon-based radical optionally comprising one or more heteroatoms, said hydrocarbon-based radical being substituted with one or more groups comprising one or more phosphorus atoms;
- M or M’ representing a hydrogen atom, an alkali metal or an alkaline-earth metal;
- - - - - representing a single bond when M or M’ is H, or an ionic bond.

It is understood that when M or M’ is other than H, then M or M’ are such that the overall charge of the molecule is zero. Thus, in the case of divalent metals, M and M’ may represent the same divalent metal.

The polyphosphorus derivatives of formula (I) are linear.

The polyphosphorus derivatives of formula (II) are cyclic.

Preferably, according to a first embodiment, the polyphosphorus derivative(s) are inorganic polyphosphate compounds, preferably chosen from:

- polyphosphates, and/or hydrates thereof; and mixtures thereof, preferably of sodium and/or potassium, such as sodium hexametaphosphate (SHMP),

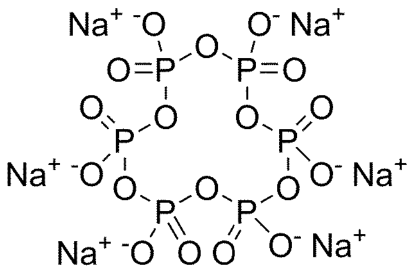

sodium polyphosphate, sodium tripolyphosphate, sodium trimetaphosphate, preferably sodium tripolyphosphate having the following formula:

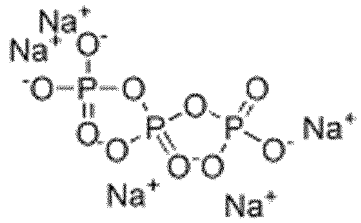

- pyrophosphates and/or hydrates thereof, and mixtures thereof, preferably of sodium and/or potassium; preferably chosen from sodium pyrophosphate and/or potassium pyrophosphate, and hydrates thereof, such as sodium pyrophosphate decahydrate having the following formula:

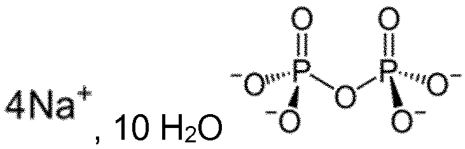

or potassium pyrophosphate having the following formula:

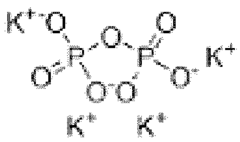

- and mixtures thereof.

According to a second embodiment, the polyphosphorus derivative(s) are organic polyphosphate derivatives and/or organic polyphosphonate derivatives, preferably chosen from polyphosphoric acids, polyphosphonic acids such as EDTMP, DETMP, ATMP, HEDP, DTPMP

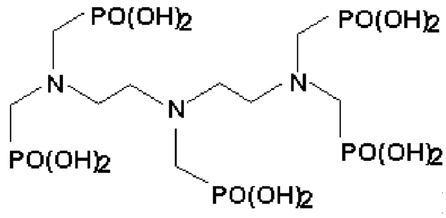

DETMP and mixtures thereof;

- iminodi(methylphosphonic) acid (example L) or salts thereof, and mixtures thereof;

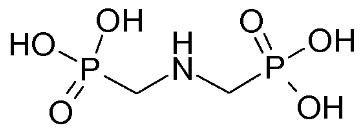

- tetrasodium etidronate (example K) of formula

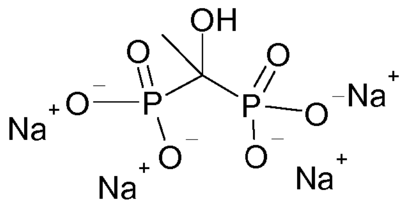

- phytic acid of formula

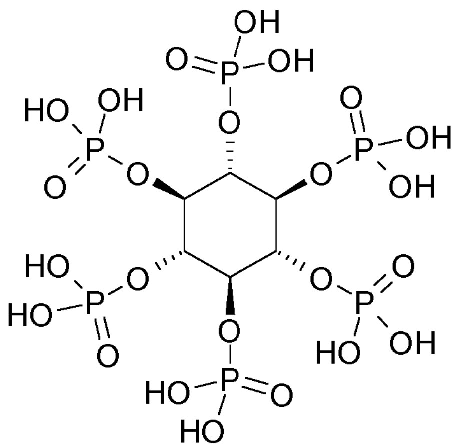

- and mixtures thereof.

Preferably, the polyphosphorus derivatives are chosen from inorganic polyphosphates, preferably chosen from: optionally hydrated alkali metal pyrophosphates, preferably chosen from sodium pyrophosphate, potassium pyrophosphate, sodium pyrophosphate decahydrate; and polymetaphosphates, such as sodium hexametaphosphate, sodium trimetaphosphate; sodium polyphosphates such as sodium tripolyphosphate, and mixtures thereof. Sodium and/or potassium is preferably used as alkali metal.

According to another embodiment, the organic polyphosphorus derivatives are chosen from polyphosphoric acids and/or salts thereof, such as phytic acid, polyphosphonic acids and/or salts thereof, such as EDTMP, DETMP, ATMP, HEDP, DTPMP, and mixtures thereof.

Preferably, the polyphosphorus derivative(s) (c) as defined previously represent a total content ranging from 0.5% to 20% by weight relative to the weight of the composition containing them, more particularly from 0.55% to 15% by weight and more preferentially from 0.7% to 12% by weight.

Particularly preferably, the polyphosphorus derivative(s) (c) as defined previously are present in a total content

ranging from 1% to 10% by weight relative to the weight of the composition.

Preferably, the polyphosphorus derivative(s) (c) as defined previously represent a total content ranging from 2% to 10% by weight and preferably from 2.5% to 10% by weight, relative to the total weight of the composition.

The composition according to the invention may also comprise one or more additional basifying agents other than the carbonate(s) (b) as defined previously, and other than the polyphosphorus derivative(s) (c) as defined previously.

***Additional basifying agent***

The additional basifying agent may be mineral or organic. It may be chosen from i) alkanolamines such as monoethanolamine, diethanolamine, triethanolamine and derivatives thereof, ii) oxyethylenated and/or oxypropylenated ethylenediamines, iii) mineral or organic hydroxides, iv) alkali metal silicates such as sodium metasilicates, v) amino acids, preferably basic amino acids such as arginine, lysine, ornithine, citrulline and histidine, and vi) the compounds of formula (II) below:

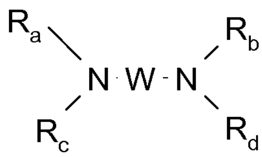
(II)

in which:

- W is a divalent ($C_1$-$C_8$)alkylene group, preferably a propylene group, optionally substituted especially with a hydroxyl group or a $C_1$-$C_4$ alkyl radical;
- $R_a$, $R_b$, $R_c$ and $R_d$, which may be identical or different, represent a hydrogen atom or a $C_1$-$C_4$ alkyl or $C_1$-$C_4$ hydroxyalkyl radical.

The mineral or organic hydroxides are preferably chosen from i) hydroxides of an alkali metal, ii) hydroxides of an alkaline-earth metal, for instance sodium hydroxide or potassium hydroxide, iii) hydroxides of a transition metal, such as hydroxides of metals from groups III, IV, V and VI, iv) hydroxides of lanthanides or actinides.

The basifying agent(s) d) as defined previously preferably represent from 0.001% to 10% by weight of the weight of the composition(s) containing them. More particularly, from 0.005% to 8% of the weight of the composition.

According to an advantageous embodiment of the invention, the composition according to the invention does not comprise any ammonia or any ammonia-generating compound, such as ammonium salts. According to a particularly preferred embodiment, the composition does not comprise any ammonium salts. Thus, preferably, the alkaline agent is not chosen from ammonia or ammonium salts.

According to an advantageous embodiment of the invention, the lightening composition according to the invention does not comprise any aqueous ammonia.

According to an advantageous embodiment of the invention, the lightening composition according to the invention comprises little or no organic amines such as alkanolamines and in particular such as monoethanolamine.

Preferably, the lightening composition according to the invention does not comprise any additional amino alkaline agent, in particular no ammonia or organic amine.

The composition according to the invention may also comprise one or more acidifying agents other than the polyphosphorus derivatives described previously.

Among the acidifying agents, examples that may be mentioned include mineral acids, for instance hydrochloric acid, (ortho)phosphoric acid, boric acid, nitric acid or sulfuric acid, or organic acids, for instance compounds comprising at least one carboxylic acid function such as acetic acid, tartaric acid, citric acid or lactic acid, a sulfonic acid function, a phosphonic acid function or a phosphoric acid function.

Preferably, citric acid is used.

The pH may be adjusted by adding acidifying agents which may be chosen from hydrochloric acid, (ortho) phosphoric acid, sulfuric acid, boric acid, and also carboxylic acids, for instance acetic acid, lactic acid or citric acid, or sulfonic acids.

*pH of the composition*

The lightening composition according to the invention has a pH of less than or equal to 9.7.

The lightening composition according to the invention preferably has a pH of less than or equal to 9.5.

Preferably, the composition according to the invention has a pH ranging from 7 to 9.7.

Preferably, the pH of the composition according to the invention ranges from 7 to 9.5, preferably from 7 to 8.7.

In particular, it is observed that, relative to the existing lightening compositions, the compositions according to the invention make it possible to obtain a good or a better level of lightening, while at the same time having a substantially lower pH (i.e. closer to 7).

***Fatty substances***

According to one embodiment, the composition according to the invention may comprise one or more fatty substances.

For the purposes of the present invention, the term "fatty substance" means an organic compound that is insoluble in water at ordinary room temperature (20-25°C) and at atmospheric pressure (760 mmHg, i.e. $1.013 \times 10^5$ Pa), with a solubility in water of less than 5%, preferably less than 1% and even more preferentially less than 0.1%. The fatty substances generally have in their structure a hydrocarbon-based chain comprising at least 6 carbon atoms. In addition, the fatty substances are generally soluble in organic solvents under the same temperature and pressure conditions, for instance chloroform, ethanol, benzene, liquid petroleum jelly or decamethylcyclopentasiloxane.

The fatty substances are, moreover, non-(poly)oxyalkylenated and non-(poly)glycerolated. In other words, the fatty substances do not comprise in their structure a (poly)ethylene oxide or (poly)glycerol or (poly)propylene glycol unit.

The fatty substance(s) may be chosen from solid fatty substances and/or liquid fatty substances (also called "oil"), and mixtures thereof.

The term *"oil"* means a *"fatty substance"* which is liquid, i.e. which is capable of flowing under the action of its own weight at room temperature (25°C) and at atmospheric pressure (760 mmHg, i.e. $1,013 \times 10^5$ Pa). Preferably, the viscosity at a temperature of 25°C and at a shear rate of 1 $s^{-1}$ of the oil is between $10^{-3}$ Pa.s and 2 Pa.s. It may be measured using a Thermo Haake RS600 rheometer with cone-plate geometry or an equivalent machine.

For the purposes of the present invention, the term "solid fatty substance" means a fatty substance that is not liquid at room temperature (20-25°C) and atmospheric pressure (760 mmHg, i.e. $1.013 \times 10^5$ Pa), in particular a solid compound or a compound having a viscosity of greater than 2 Pa.s at a shear rate of 1 $s^{-1}$ under the conditions mentioned above.

The solid fatty substances used in the composition according to the invention have a melting point above room temperature, preferably a melting point greater than or equal to 40°C, preferentially ranging from 46 to 95°C.

In particular, the fatty substance may be chosen from hydrocarbon-based fatty substances, silicone fatty substances and/or fluoro fatty substances.

The term "hydrocarbon-based fatty substance" means a fatty substance formed essentially of, or even constituted of, carbon and hydrogen atoms, and optionally of oxygen or nitrogen atoms, and not comprising any silicon or fluorine atoms. It may contain alcohol, ester, ether, carboxylic acid, amine and/or amide groups.

The hydrocarbon-based fatty substance may be chosen in particular from hydrocarbons, fatty substances of animal origin, fatty substances of plant origin, fatty alcohols, fatty esters and fatty ethers.

According to a first embodiment, the fatty substance may be a silicone fatty substance.

The term *"silicone* fatty substance" means a fatty substance containing at least one silicon atom. The term *"non-silicone* fatty substance" means a fatty substance not containing any silicon (Si) atoms.

According to one embodiment, the silicone fatty substance may be a liquid silicone oil (also known as silicone oil or liquid silicone). The term "liquid silicone" means an organopolysiloxane that is liquid at ordinary temperature (25°C) and at atmospheric pressure (760 mmHg; i.e. $1.013 \times 10^5$ Pa).

Preferably, the silicone is chosen from liquid polydialkylsiloxanes, in particular liquid polydimethylsiloxanes (PDMSs), and liquid polyorganosiloxanes comprising at least one aryl group.

The polydialkylsiloxanes are chosen in particular from polydimethylsiloxanes comprising trimethylsilyl end groups, and polydimethylsiloxanes comprising dimethylsilanol end groups, known under the name dimethiconol (CTFA). The polyorganosiloxanes comprising aryl groups are chosen in particular from polydiarylsiloxanes, in particular polydiphenylsiloxanes, and polyalkylarylsiloxanes.

Organopolysiloxanes are defined in greater detail in Walter Noll's Chemistry and Technology of Silicones (1968), Academic Press. They may be volatile or non-volatile.

When they are volatile, the silicones are more particularly chosen from those with a boiling point of between 60°C and 260°C, and even more particularly from:

(i) cyclic polydialkylsiloxanes comprising from 3 to 7 and preferably from 4 to 5 silicon atoms. These are, for example, octamethylcyclotetrasiloxane sold in particular under the name Volatile Silicone® 7207 by Union Carbide or Silbione® 70045 V2 by Rhodia, decamethylcyclopentasiloxane sold under the name Volatile Silicone® 7158 by Union Carbide, and Silbione® 70045 V5 by Rhodia, and mixtures thereof.

Mention may also be made of cyclocopolymers of the dimethylsiloxane/methylalkylsiloxane type, such as

Volatile Silicone® FZ 3109 sold by Union Carbide, having the formula:

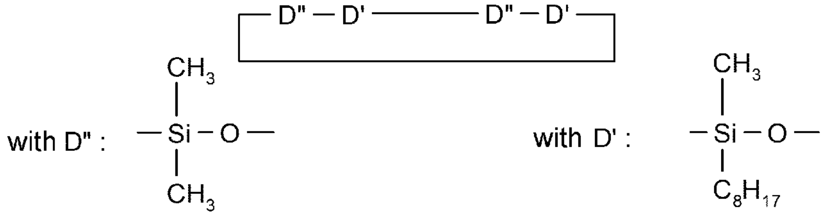

Mention may also be made of mixtures of cyclic polydialkylsiloxanes with organosilicon compounds, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane;

(ii) linear volatile polydialkylsiloxanes containing 2 to 9 silicon atoms and having a viscosity of less than or equal to $5\times10^{-6}$ $m^2/s$ at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, "Volatile Silicone Fluids for Cosmetics".

Non-volatile polydialkylsiloxanes, polydialkylsiloxane gums and resins, polyorganosiloxanes modified with the above organofunctional groups, and mixtures thereof, are preferably used.

These silicones are more particularly chosen from polydialkylsiloxanes, among which mention may be made mainly of polydimethylsiloxanes bearing trimethylsilyl end groups. The viscosity of the silicones is measured at 25°C according to ASTM Standard 445 Appendix C.

Among these polydialkylsiloxanes, mention may be made, in a nonlimiting manner, of the following commercial products:

- the Silbione® oils of the 47 and 70 047 series or the Mirasil® oils sold by Rhodia, for instance the oil 70 047 V 500 000;
- the oils of the Mirasil® series sold by Rhodia;
- the oils of the 200 series from the company Dow Corning, such as DC200 with a viscosity of 60 000 $mm^2/s$;
- the Viscasil® oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.

In this category of polydialkylsiloxanes, mention may also be made of the products sold under the names Abil Wax® 9800 and 9801 by the company Goldschmidt, which are polydi($C_1$-$C_{20}$)alkylsiloxanes.

The silicone gums that may be used in accordance with the invention are especially polydialkylsiloxanes and preferably polydimethylsiloxanes with high number-average molecular masses of between 200 000 and 1 000 000, used alone or as a mixture in a solvent. This solvent may be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Preferably, the fatty substances that may be used in the composition according to the invention are non-silicone fatty substances, i.e. a fatty substance not containing any silicon (Si) atoms.

According to a second embodiment, the fatty substance may be a fluoro fatty substance. The term "fluoro fatty substance" means a fatty substance containing at least one fluorine atom.

In particular, fluoro fatty substances that may be mentioned include fluoro oils, for instance perfluoromethylcyclopentane and perfluoro-1,3-dimethylcyclohexane, sold under the names Flutec® PC1 and Flutec® PC3 by the company BNFL Fluorochemicals; perfluoro-1,2-dimethylcyclobutane; perfluoroalkanes such as dodecafluoropentane and tetradecafluorohexane, sold under the names PF 5050® and PF 5060® by the company 3M, or bromoperfluorooctyl sold under the name Foralkyl® by the company Atochem; nonafluoromethoxybutane and nonafluoroethoxyisobutane; perfluoromorpholine derivatives such as 4-trifluoromethylperfluoromorpholine sold under the name PF 5052® by the company 3M.

According to a preferred third embodiment, the fatty substance is a hydrocarbon-based fatty substance as defined above. In particular, preferably, the hydrocarbon-based fatty substance(s) are chosen from hydrocarbon-based oils and hydrocarbon-based solid fatty substances, and mixtures thereof, preferably chosen from hydrocarbon-based oils.

Preferably, the fatty substance(s), which are preferably hydrocarbon-based, are advantageously chosen from linear, branched, optionally cyclic, $C_6$-$C_{16}$ alkanes, such as hexane, dodecane, isoparaffins such as isohexadecane, isodecane, isododecane, and mixtures thereof; hydrocarbons containing more than 16 carbon atoms, preferably liquid paraffins, petroleum jelly, liquid petroleum jelly, polydecenes, and hydrogenated polyisobutene such as Parleam®, and mixtures thereof, liquid fatty alcohols such as octyldodecanol, fatty acids, and liquid esters of fatty acids and/or of fatty alcohols, or mixtures thereof.

According to a first preferred variant of the invention, the fatty substance is an oil, preferably a hydrocarbon-based oil.

Preferably, the hydrocarbon-based oils are chosen from:

- halogenated or non-halogenated, linear or branched hydrocarbons, of mineral or synthetic origin, containing less than 16 carbon atoms, in particular containing between 6 and 15 carbon atoms, for instance hexane, cyclohexane, undecane, dodecane, isododecane, isohexadecane or tridecane, or containing more than 16 carbon atoms, such as liquid petroleum jelly, liquid paraffin, polydecenes of formula $C_{10n}H_{[(20n)+2]}$ in which n ranges from 3 to 9 and preferably from 3 to 7, polybutenes, hydrogenated polybutenes, polyisobutene, hydrogenated polyisobutenes such as Parleam®, and mixtures thereof;
- unsaturated or branched liquid fatty alcohols containing from 6 to 30 carbon atoms, such as those of formula $C_nH_{2n+1}OH$ with n being an integer between 6 and 20 inclusive. Mention may be made especially of oleyl alcohol, linolenyl alcohol, linoleyl alcohol, ricinoleyl alcohol, undecylenyl alcohol, isostearyl alcohol and octyldodecanol;
- triglyceride oils of plant or synthetic origin, such as liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stéarineries Dubois or those sold under the names Miglyol® 810, 812 and 818 by the company Dynamit Nobel, jojoba oil and shea butter oil; and
- liquid esters other than triglycerides.

These esters are preferably liquid esters of saturated or unsaturated, linear or branched $C_1$-$C_{26}$ aliphatic monoacids or polyacids and of saturated or unsaturated, linear or branched $C_1$-$C_{26}$ aliphatic monoalcohols or polyalcohols, the total number of carbon atoms of the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, 2-ethylhexyl isononanoate, isodecyl neopentanoate and isostearyl neopentanoate.

Esters of $C_4$-$C_{22}$ dicarboxylic or tricarboxylic acids and of $C_1$-$C_{22}$ alcohols and esters of monocarboxylic, dicarboxylic or tricarboxylic acids and of non-sugar $C_4$-$C_{26}$ dihydroxy, trihydroxy, tetrahydroxy or pentahydroxy alcohols may also be used.

Mention may be made especially of: diethyl sebacate; diisopropyl sebacate; diisopropyl adipate; di-n-propyl adipate; dioctyl adipate; diisostearyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; triisostearyl citrate; glyceryl trilactate; glyceryl trioctanoate; trioctyldodecyl citrate; trioleyl citrate; propylene glycol dioctanoate; neopentyl glycol diheptanoate; diethylene glycol diisononanoate; and polyethylene glycol distearates.

Among the esters mentioned above, it is preferred to use ethyl, isopropyl, myristyl, cetyl or stearyl palmitate, 2-ethylhexyl palmitate, 2-octyldecyl palmitate, alkyl myristates such as isopropyl, butyl, cetyl or 2-octyldodecyl myristate, hexyl stearate, propylene glycol dicaprylate, butyl stearate, isobutyl stearate; dioctyl malate, hexyl laurate, 2-hexyldecyl laurate, isononyl isononanoate or cetyl octanoate.

The composition may also comprise, as liquid fatty ester, sugar esters and diesters of $C_6$-$C_{30}$ and preferably $C_{12}$-$C_{22}$ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligo saccharides or polysaccharides.

The hydrocarbon-based oils are preferably chosen from the polydecenes of formula $C_{10n}H_{[(20n)+2]}$ in which n ranges from 3 to 9 and preferably from 3 to 7, fatty alcohols, esters and in particular esters of fatty alcohols or of fatty acids, sugar esters or diesters of $C_{12}$-$C_{24}$ fatty acids, cyclic esters, cyclic ethers, hydrocarbon-based oils, mineral oils, plant oils or animal oils, or mixtures thereof.

Preferably, the liquid fatty substance(s) are chosen from polydecenes, liquid petroleum jelly, liquid paraffin, isododecane, fatty alcohols such as octyldodecanol or isostearyl alcohol, and liquid esters of fatty alcohols or of fatty acids, and mixtures thereof.

Even more preferentially, the liquid fatty substances are chosen from liquid petroleum jelly, isododecane, octyldodecanol, and mixtures thereof.

Preferably, the liquid fatty substance(s) (that are liquid at room temperature), which are preferably hydrocarbon-based, are present in the composition according to the invention in a total content ranging from 5% to 80% by weight, more preferentially from 10% to 80% by weight, preferably from 15% to 75% by weight, better still from 20% to 70% by weight,

even more advantageously from 25% to 70% and preferentially from 25% to 60% by weight relative to the total weight of the composition.

According to a second variant of the invention, the hydrocarbon-based fatty substance is solid. The composition according to the invention thus comprises one or more solid, hydrocarbon-based fatty substances.

Preferably, the solid fatty substance(s) are chosen from fatty alcohols, and esters of fatty acids and/or of fatty alcohols and also mixtures thereof.

According to a preferred embodiment, the solid hydrocarbon-based fatty substance(s) are chosen from solid fatty alcohols and/or solid esters of fatty acids and/or of fatty alcohols. Preferably, the solid hydrocarbon-based fatty substances are chosen from linear or branched, saturated or unsaturated solid fatty alcohols comprising from 14 to 30 carbon atoms and/or solid esters derived from $C_9$-$C_{26}$ fatty acids and from $C_9$-$C_{26}$ fatty alcohols.

Preferably, the solid fatty alcohols are saturated or unsaturated, and linear or branched, and comprise from 14 to 30 carbon atoms. Preferably, the fatty alcohol(s) are chosen from saturated and linear fatty alcohols comprising from 14 to 30 and preferably from 16 to 22 carbon atoms.

In addition, it is understood that the fatty alcohols do not comprise any $C_2$-$C_3$ oxyalkylene units or any glycerol units.

In a preferred manner, the solid hydrocarbon-based fatty substances are chosen from cetyl alcohol, stearyl alcohol, behenyl alcohol and a mixture thereof. Use may be made, for example, of cetylstearyl alcohol.

The hydrocarbon-based solid fatty substance(s) may also be chosen from solid esters of fatty acids and/or of of fatty alcohols; mention may be made especially of the solid esters derived from $C_9$-$C_{26}$ fatty acids and from $C_9$-$C_{26}$ fatty alcohols.

In particular, these esters may be chosen from octyldodecyl behenate, isocetyl behenate, cetyl lactate, stearyl octanoate, octyl octanoate, cetyl octanoate, decyl oleate, myristyl stearate, octyl palmitate, cetyl palmitate, octyl pelargonate, octyl stearate, alkyl myristates such as cetyl myristate, myristyl myristate or stearyl myristate, and hexyl stearate.

Preferably, the fatty substance(s) that are solid at room temperature, which are preferably hydrocarbon-based, are present in the composition according to the invention in a total content ranging from 0.1% to 15% by weight, preferably from 0.5% to 10% by weight and more preferentially from 1% to 8% by weight relative to the total weight of the composition.

Preferably, the fatty substance(s) are chosen from hydrocarbon-based fatty substances, more preferentially from:

- linear, branched, optionally cyclic $C_6$-$C_{16}$ alkanes, such as hexane, dodecane, isoparaffins such as isohexadecane, isodecane, and mixtures thereof;
- hydrocarbons with more than 16 carbon atoms, preferably liquid paraffins, petroleum jelly, liquid petroleum jelly, polydecenes, and hydrogenated polyisobutene such as Parleam®, and mixtures thereof;
- fatty alcohols, such as octyldodecanol, isostearyl alcohol, cetyl alcohol, stearyl alcohol and behenyl alcohol, and mixtures thereof;
- and mixtures thereof.

Preferably, they are chosen from hydrocarbon-based liquid fatty substances.

The composition according to the invention may preferably comprise at least 10% by total weight of fatty substances, which are preferably non-silicone, in particular of oils, which are preferably hydrocarbon-based, relative to the total weight of the composition of the invention, preferably at least 10%.

More particularly, the composition according to the invention may comprise at least 15% by weight, better still at least 20% by weight and even better still at least 25% by weight of fatty substances, which are preferably non-silicone, in particular of oils, preferably non-silicone oils, relative to the total weight of the composition.

The composition according to the invention more particularly has a content of fatty substances, which are preferably non-silicone, in particular of oils, preferably non-silicone oils, in a total content ranging from 5% to 80% by weight, more preferentially from 10% to 80% by weight, preferably from 15% to 75% by weight, better still from 20% to to 70% by weight, even more advantageously from 25% to 70% and preferentially from 25% to 60% by weight relative to the total weight of the composition.

***Surfactants:***

The composition according to the present invention may comprise one or more surfactants. These surfactants may be chosen from anionic surfactants, amphoteric or zwitterionic surfactants, nonionic surfactants and cationic surfactants.

The term "anionic surfactant" means a surfactant comprising, as ionic or ionizable groups, only anionic groups. These anionic groups are preferably chosen from the groups $CO_2H$, $CO_2^-$, $SO_3H$, $SO_3^-$, $OSO_3H$, $OSO_3^-$, $H_2PO_3$, $HPO_3^-$,

$PO_3^{2-}$, $H_2PO_2$, $HPO_2^-$, $PO_2^{2-}$, POH and $PO^-$.

As examples of anionic surfactants that can be used in the composition according to the invention, mention may be made of alkyl sulfates, alkyl ether sulfates, alkylamido ether sulfates, alkylaryl polyether sulfates, monoglyceride sulfates, alkyl sulfonates, alkylamide sulfonates, alkylaryl sulfonates, α-olefin sulfonates, paraffin sulfonates, alkyl sulfosuccinates, alkyl ether sulfosuccinates, alkylamide sulfosuccinates, alkyl sulfoacetates, acyl sarcosinates, acyl glutamates, alkyl sulfosuccinamates, acyl isethionates and N-($C_1$-$C_4$)alkyl N-acyl taurates, salts of alkyl monoesters of polyglycoside-polycarboxylic acids, acyl lactylates, salts of D-galactoside uronic acids, salts of alkyl ether carboxylic acids, salts of alkylaryl ether carboxylic acids, salts of alkylamido ether carboxylic acids; and the corresponding non-salified forms of all these compounds; the alkyl and acyl groups of all these compounds (unless specified otherwise) generally comprising from 6 to 24 carbon atoms and the aryl group generally denoting a phenyl group.

These compounds may be oxyethylenated and then preferably comprise from 1 to 50 ethylene oxide units.

The salts of $C_6$-$C_{24}$ alkyl monoesters of polyglycoside-polycarboxylic acids may be chosen from $C_6$-$C_{24}$ alkyl polyglycoside-citrates, $C_6$-$C_{24}$ alkyl polyglycoside-tartrates and $C_6$-$C_{24}$ alkyl polyglycoside-sulfosuccinates.

When the anionic surfactant(s) are in salt form, they may be chosen from alkali metal salts such as the sodium or potassium salt and preferably the sodium salt, ammonium salts, amine salts and in particular amino alcohol salts or alkaline-earth metal salts such as the magnesium salt.

Examples of amino alcohol salts that may especially be mentioned include the salts of mono-, di- and triethanolamine, the salts of mono-, di- or triisopropanolamine, and the salts of 2-amino-2-methyl-1-propanol, of 2-amino-2-methyl-1,3-propanediol and of tris(hydroxymethyl)aminomethane.

Alkali metal or alkaline-earth metal salts and in particular the sodium or magnesium salts are preferably used.

The anionic surfactants optionally present may be mild anionic surfactants, i.e. anionic surfactants without a sulfate function.

As regards the mild anionic surfactants, mention may be made in particular of the following compounds and salts thereof, and also mixtures thereof:

- polyoxyalkylenated alkyl ether carboxylic acids;
- polyoxyalkylenated alkylaryl ether carboxylic acids;
- polyoxyalkylenated alkylamido ether carboxylic acids, in particular those comprising from 2 to 50 ethylene oxide groups;
- alkyl-D-galactoside uronic acids;
- acyl sarcosinates, acyl glutamates; and
- alkyl polyglycoside carboxylic esters.

Use may be made most particularly of polyoxyalkylenated alkyl ether carboxylic acids, for instance lauryl ether carboxylic acid (4.5 EO), sold, for example, under the name Akypo RLM 45 CA from Kao.

The amphoteric or zwitterionic surfactant(s) that may be used in the present invention may especially be optionally quaternized secondary or tertiary aliphatic amine derivatives, in which the aliphatic group is a linear or branched chain containing from 8 to 22 carbon atoms, said amine derivatives containing at least one anionic group, for instance a carboxylate, sulfonate, sulfate, phosphate or phosphonate group.

Mention may be made in particular of ($C_8$-$C_{20}$)alkylbetaines, sulfobetaines, ($C_8$-$C_{20}$)alkylamido($C_3$-$C_8$)alkylbetaines or ($C_8$-$C_{20}$)alkylamido-($C_6$-$C_8$)-alkylsulfobetaines.

Among the optionally quaternized secondary or tertiary aliphatic amine derivatives that may be used, as defined above, mention may also be made of the compounds of respective structures ($A_1$), ($A_2$) and ($A_3$) below:

$$R_a\text{-}CONHCH_2CH_2\text{-}N^+(R_b)(R_c)(CH_2COO^-) \qquad (A_1)$$

in which:

- $R_a$ represents a $C_{10}$-$C_{30}$ alkyl or alkenyl group derived from an acid $R_a$-COOH preferably present in hydrolysed coconut oil, or a heptyl, nonyl or undecyl group,
- $R_b$ represents a β-hydroxyethyl group, and
- $R_c$ represents a carboxymethyl group,
- and

$$R'_a\text{-}CONHCH_2CH_2\text{-}N(B)(B') \qquad (A_2)$$

in which:

B represents $-CH_2CH_2OX'$,
B' represents $-(CH_2)_z-Y'$, with z = 1 or 2,
X' represents the group $-CH_2-COOH$, $CH_2-COOZ'$, $-CH_2CH_2-COOH$ or $-CH_2CH_2-COOZ'$, or a hydrogen atom,
Y' represents -COOH, -COOZ', or the group $CH_2-CHOH-SO_3H$ or $-CH_2-CHOH-SO_3Z'$,
Z' represents an ion derived from an alkali metal or alkaline-earth metal, such as sodium, an ammonium ion or an ion derived from an organic amine,
$R'_a$ represents a $C_{10}$-$C_{30}$ alkyl or alkenyl group of an acid $R'_a$-COOH preferably present in hydrolysed linseed oil or coconut oil, an alkyl group, in particular a $C_{17}$ alkyl group and its iso form, or an unsaturated $C_{17}$ group.

These compounds of formula ($A_1$) or ($A_2$) are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium caprylamphodipropionate, lauroamphodipropionic acid, cocoamphodipropionic acid.
By way of example, mention may be made of the cocoamphodiacetate sold by the company Rhodia under the trade name Miranol® C2M Concentrate.

$$R_a''\text{-NHCH(Y'')-}(CH_2)_n\text{CONH}(CH_2)n'\text{-N}(R_d)(R_e) \qquad (A_3)$$

in which formula:

Y'' represents the group -COOH, -COOZ'' or $-CH_2-CH(OH)SO_3H$ or the group $-CH_2CH(OH)SO_3$-Z'';
$R_d$ and $R_e$, independently of each other, represent a $C_1$-$C_4$ alkyl or hydroxyalkyl radical;
Z'' represents a cationic counterion derived from an alkali metal or alkaline-earth metal, such as sodium, an ammonium ion or an ion derived from an organic amine;
$R_a''$ represents a $C_{10}$-$C_{30}$ alkyl or alkenyl group of an acid $R_a''$-COOH preferably present in hydrolysed linseed oil or coconut oil;
n and n' denote, independently of each other, an integer ranging from 1 to 3.

Among the compounds of formula ($A_3$), mention may be made of the compound classified in the CTFA dictionary under the name sodium diethylaminopropyl cocoaspartamide and sold by the company Chimex under the name Chimexane HB.
Among the abovementioned amphoteric or zwitterionic surfactants, it is preferred to use ($C_8$-$C_{20}$ alkyl)betaines such as cocoylbetaine, ($C_8$-$C_{20}$ alkyl)amido($C_3$-$C_8$ alkyl)betaines such as cocoylamidopropylbetaine, and mixtures thereof. More preferentially, the amphoteric or zwitterionic surfactant(s) are chosen from cocoylamidopropylbetaine and cocoylbetaine.
The nonionic surfactant(s) in the compositions of the present invention are especially described, for example, in the Handbook of Surfactants by M.R. Porter, published by Blackie & Son (Glasgow and London), 1991, pages 116-178. They are especially chosen from fatty alcohols, fatty $\alpha$-diols, fatty ($C_1$-$C_{20}$)alkylphenols and fatty acids, these compounds being ethoxylated, propoxylated or glycerolated and containing at least one fatty chain comprising, for example, from 8 to 18 carbon atoms, the number of ethylene oxide or propylene oxide groups possibly ranging especially from 1 to 200, and the number of glycerol groups possibly ranging especially from 1 to 30.
Mention may also be made of condensates of ethylene oxide and of propylene oxide with fatty alcohols, ethoxylated fatty amides preferably containing from 1 to 30 ethylene oxide units, polyglycerolated fatty amides comprising on average from 1 to 5 glycerol groups, and in particular from 1.5 to 4, ethoxylated fatty acid esters of sorbitan containing from 1 to 30 ethylene oxide units, fatty acid esters of sucrose, fatty acid esters of polyethylene glycol, ($C_6$-$C_{24}$ alkyl) polyglycosides, oxyethylenated plant oils, N-($C_6$-$C_{24}$ alkyl)glucamine derivatives, amine oxides such as ($C_{10}$-$C_{14}$ alkyl) amine oxides or N-($C_{10}$-$C_{14}$ acyl)aminopropylmorpholine oxides.
The cationic surfactant(s) that may be used in the composition according to the invention are generally chosen from optionally polyoxyalkylenated primary, secondary or tertiary fatty amine salts, quaternary ammonium salts, and mixtures thereof.
Examples of quaternary ammonium salts that may especially be mentioned include:

- those corresponding to the general formula (X) below:

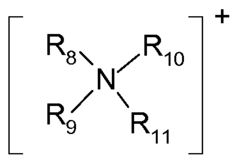

(X)

in which the groups $R_8$ to $R_{11}$, which may be identical or different, represent a linear or branched aliphatic group comprising from 1 to 30 carbon atoms or an aromatic group, such as aryl or alkylaryl, at least one of the groups $R_8$ to $R_{11}$ comprising from 8 to 30 carbon atoms and preferably from 12 to 24 carbon atoms. The aliphatic groups may comprise heteroatoms especially such as oxygen, nitrogen, sulfur and halogens.

The aliphatic groups are chosen, for example, from $C_1$-$C_{30}$ alkyl, $C_1$-$C_{30}$ alkoxy, polyoxy($C_2$-$C_6$)alkylene, $C_1$-$C_{30}$ alkylamide, ($C_{12}$-$C_{22}$)alkylamido($C_2$-$C_6$)alkyl, ($C_{12}$-$C_{22}$)alkyl acetate and $C_1$-$C_{30}$ hydroxyalkyl groups; $X^-$ is an anion chosen from the group of the halides, phosphates, acetates, lactates, ($C_1$-$C_4$)alkyl sulfates and ($C_1$-$C_4$)alkyl- or ($C_1$-$C_4$) alkylarylsulfonates.

Among the quaternary ammonium salts of formula (X), preference is given, firstly, to tetraalkylammonium chlorides, for instance dialkyldimethylammonium or alkyltrimethylammonium chlorides in which the alkyl group comprises from about 12 to 22 carbon atoms, in particular behenyltrimethylammonium chloride, distearyldimethylammonium chloride, cetyltrimethylammonium chloride or benzyldimethylstearylammonium chloride, or else, secondly, to distearoylethylhydroxyethylmethylammonium methosulfate, dipalmitoylethylhydroxyethylammonium methosulfate or distearoylethylhydroxyethylammonium methosulfate, or else, finally, to palmitylamidopropyltrimethylammonium chloride or stearamidopropyldimethyl(myristyl acetate)ammonium chloride, sold under the name Ceraphyl® 70 by the company Van Dyk;

- quaternary ammonium salts of imidazoline, for instance those of formula (XI) below:

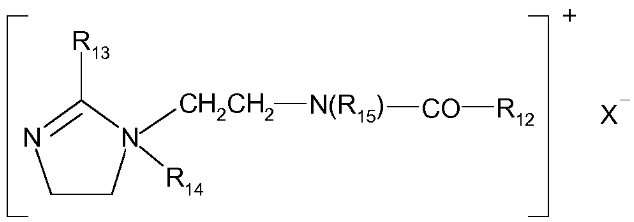

(XI)

in which

$R_{12}$ represents an alkenyl or alkyl group comprising from 8 to 30 carbon atoms, for example derived from tallow fatty acids,
$R_{13}$ represents a hydrogen atom, a $C_1$-$C_4$ alkyl group or an alkenyl or alkyl group comprising from 8 to 30 carbon atoms,
$R_{14}$ represents a $C_1$-$C_4$ alkyl group,
$R_{15}$ represents a hydrogen atom, a $C_1$-$C_4$ alkyl group, $X^-$ is an anion chosen from the group of halides, phosphates, acetates, lactates, ($C_1$-$C_4$)alkyl sulfates and ($C_1$-$C_4$)alkylsulfonates or ($C_1$-$C_4$)alkylarylsulfonates.

Preferably, $R_{12}$ and $R_{13}$ denote a mixture of alkenyl or alkyl groups comprising from 12 to 21 carbon atoms, for example derived from tallow fatty acids, $R_{14}$ denotes a methyl group and $R_{15}$ denotes a hydrogen atom. Such a product is sold, for example, under the name Rewoquat® W 75 by the company Rewo.

- quaternary diammonium or triammonium salts, particularly of formula (XII) below:

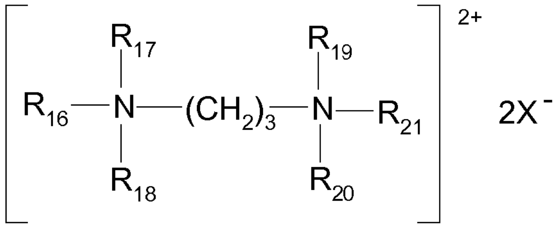

(XII)

in which $R_{16}$ denotes an alkyl group comprising approximately from 16 to 30 carbon atoms, which is optionally hydroxylated and/or interrupted with one or more oxygen atoms,

$R_{17}$ is chosen from hydrogen, an alkyl group comprising from 1 to 4 carbon atoms or a group $-(CH_2)_3-N^+(R_{16a})(R_{17a})(R_{18a})$,
$R_{16a}$, $R_{17a}$, $R_{18a}$, $R_{18}$, $R_{19}$, $R_{20}$ and $R_{21}$, which may be identical or different, are chosen from hydrogen or an alkyl group comprising from 1 to 4 carbon atoms, and
$X^-$ is an anion chosen from the group of halides, acetates, phosphates, nitrates, $(C_1-C_4)$alkyl sulfates, $(C_1-C_4)$ alkyl- or $(C_1-C_4)$alkylarylsulfonates, in particular methyl sulfate and ethyl sulfate.

Such compounds are, for example, Finquat CT-P, available from Finetex (Quaternium 89), and Finquat CT, available from Finetex (Quaternium 75);

- quaternary ammonium salts comprising one or more ester functions, such as those of formula (XIII) below:

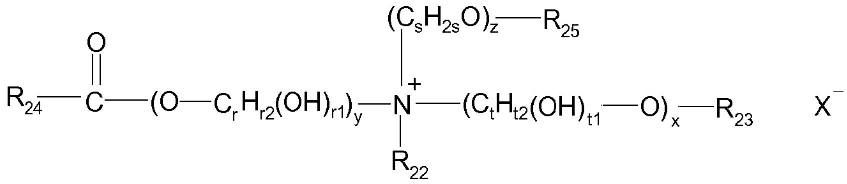

(XIII)

in which:

$R_{22}$ is chosen from $C_1-C_6$ alkyl groups and $C_1-C_6$ hydroxyalkyl or dihydroxyalkyl groups,
$R_{23}$ is chosen from:

- the group

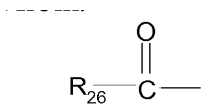

,

- saturated or unsaturated, linear or branched $C_1-C_{22}$ hydrocarbon-based groups $R_{27}$,
- a hydrogen atom,

$R_{25}$ is chosen from:

- the group

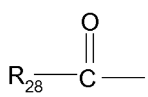

,

- saturated or unsaturated, linear or branched $C_1-C_6$ hydrocarbon-based groups $R_{29}$,
- a hydrogen atom,

$R_{24}$, $R_{26}$ and $R_{28}$, which may be identical or different, are chosen from saturated or unsaturated, linear or branched $C_7$-$C_{21}$ hydrocarbon-based groups;
r, s and t, which may be identical or different, are integers ranging from 2 to 6,
r1 and t1, which may be identical or different, are equal to 0 or 1,
$r2 + r1 = 2\ r$ and $t1 + t2 = 2\ t$,
y is an integer ranging from 1 to 10,
x and z, which may be identical or different, are integers ranging from 0 to 10,
$X^-$ is a simple or complex, organic or inorganic anion,
with the proviso that the sum x + y + z is from 1 to 15, that when x is 0 then $R_{23}$ denotes $R_{27}$, and that when z is 0 then $R_{25}$ denotes $R_{29}$.

The alkyl groups $R_{22}$ may be linear or branched, and more particularly linear.
Preferably, $R_{22}$ denotes a methyl, ethyl, hydroxyethyl or dihydroxypropyl group, and more particularly a methyl or ethyl group.
Advantageously, the sum x + y + z ranges from 1 to 10.
When $R_{23}$ is a hydrocarbon-based group $R_{27}$, it may be long and contain from 12 to 22 carbon atoms, or may be short and contain from 1 to 3 carbon atoms.
When $R_{25}$ is a hydrocarbon-based group $R_{29}$, it preferably contains 1 to 3 carbon atoms.
Advantageously, $R_{24}$, $R_{26}$ and $R_{28}$, which may be identical or different, are chosen from linear or branched, saturated or unsaturated $C_{11}$-$C_{21}$ hydrocarbon-based groups, and more particularly from linear or branched, saturated or unsaturated $C_{11}$-$C_{21}$ alkyl and alkenyl groups.
Preferably, x and z, which may be identical or different, are equal to 0 or 1.
Advantageously, y is equal to 1.
Preferably, r, s and t, which may be identical or different, are equal to 2 or 3, and even more particularly are equal to 2.
The anion $X^-$ is preferably a halide, preferably chloride, bromide or iodide, a $(C_1$-$C_4)$alkyl sulfate, or a $(C_1$-$C_4)$alkylsulfonate or $(C_1$-$C_4)$alkylarylsulfonate. However, use may be made of methanesulfonate, phosphate, nitrate, tosylate, an anion derived from an organic acid, such as acetate or lactate, or any other anion that is compatible with the ammonium bearing an ester function.
The anion $X^-$ is even more particularly chloride, methyl sulfate or ethyl sulfate.
Use is made more particularly, in the composition according to the invention, of the ammonium salts of formula (XIII) in which:

- $R_{22}$ denotes a methyl or ethyl group,
- x and y are equal to 1,
- z is equal to 0 or 1,
- r, s and t are equal to 2,
- $R_{23}$ is chosen from:
- the group

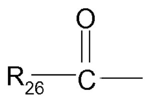

,

- methyl, ethyl or $C_{14}$-$C_{22}$ hydrocarbon-based groups,
- a hydrogen atom,
- $R_{25}$ is chosen from:
- the group

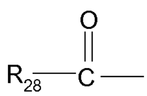

,

- a hydrogen atom,
- $R_{24}$, $R_{26}$ and $R_{28}$, which may be identical or different, are chosen from linear or branched, saturated or unsaturated $C_{13}$-$C_{17}$ hydrocarbon-based groups, and preferably from linear or branched, saturated or unsaturated $C_{13}$-$C_{17}$ alkyl and alkenyl groups.

Advantageously, the hydrocarbon-based groups are linear.

Among the compounds of formula (XIII), examples that may be mentioned include salts, especially the chloride or methyl sulfate, of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, triisopropanolamine, alkyldiethanolamine or alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent such as an alkyl halide, preferably a methyl or ethyl halide, a dialkyl sulfate, preferably a dimethyl or diethyl sulfate, methyl methanesulfonate, methyl para-toluenesulfonate, glycol chlorohydrin or glycerol chlorohydrin.

Such compounds are sold, for example, under the names Dehyquart® by the company Henkel, Stepanquat® by the company Stepan, Noxamium® by the company Ceca or Rewoquat® WE 18 by the company Rewo-Witco.

The composition according to the invention may contain, for example, a mixture of quaternary ammonium monoester, diester and triester salts with a weight majority of diester salts.

Use may also be made of the ammonium salts containing at least one ester function that are described in patents US-A-4 874 554 and US-A-4 137 180.

Use may also be made of behenoylhydroxypropyltrimethylammonium chloride, for example, sold by the company Kao under the name Quartamin BTC 131.

Preferably, the ammonium salts containing at least one ester function contain two ester functions.

Among the cationic surfactants, it is more particularly preferred to choose cetyltrimethylammonium, behenyltrimethylammonium and dipalmitoylethylhydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

Preferably, the surfactant(s) are chosen from anionic and/or nonionic surfactants, preferably nonionic surfactants.

When the composition comprises one or more surfactants, their content may preferably range from 0.05% to 20% by weight, more preferentially from 0.1% to 10% by weight and better still from 0.5% to 5% by weight relative to the total weight of the composition.

***Liquid organic solvents***

According to one embodiment of the invention, the composition according to the invention comprises one or more liquid organic solvents.

The term "organic solvent" means an organic substance which is capable of dissolving or dispersing another substance without chemically modifying it.

Said organic solvent(s) may be chosen from $C_1$-$C_4$ lower alkanols, such as ethanol and isopropanol; polyols and polyol ethers such as 2-butoxyethanol, propylene glycol, propylene glycol monomethyl ether, diethylene glycol monomethyl ether and monoethyl ether, hexylene glycol, and also aromatic alcohols such as benzyl alcohol or phenoxyethanol, and mixtures thereof.

Preferably, said organic solvent(s) are chosen from liquid organic compounds with a Hansen solubility parameter $\delta H$ of greater than 0 and less than 16 $MPa^{1/2}$.

In the context of the present invention, such a compound is also known as a "hydrotropic compound".

For the purposes of the present invention, the term "hydrotropic compound" means a compound that is capable of increasing the solubility of hydrophobic compounds in aqueous phases.

Said liquid compounds more preferentially have a Hansen solubility parameter $\delta H$ of between 5 and 15.8 MPa1/2, even more preferentially between 8 and 15.8 $MPa^{1/2}$ and better still between 8 and 15 $MPa^{1/2}$.

These compounds are liquid at a temperature of 25°C and at atmospheric pressure (760 mmHg; i.e. $1.013 \times 10^5$ Pa).

The compound(s) with a Hansen solubility parameter $\delta H$ value as defined previously are, for example, described in the reference publication Hansen solubility parameters: A User's Handbook by Charles M. Hansen, CRC Press, 2000, pages 167 to 185, or in the publication Handbook of Solubility Parameters and Other Cohesion Parameters, CRC Press, pages 95 to 121 and pages 177 to 185.

This value of the solubility parameter $\delta H$ is related to the formation of hydrogen bonds. It may be recalled that there exist three major types of interactions in organic compounds: non-polar interactions, permanent dipole-dipole interactions and interactions of hydrogen bond type, the latter interactions forming the subject of the parameter defining the

hydrotropic compound present in the composition employed in accordance with the invention.

In particular, the work Handbook of Solubility Parameters and Other Cohesion Parameters, CRC Press, pages 95 to 121 and pages 177 to 185, gives the equation $\delta H = (\sum\text{-}zUh/V)^{1/2}$ in which zUh (in $J.mol^{-1}$) describes the contributions of the functional group considered in the solubility parameters associated with the hydrogen bonds (values in Table 14, page 183), this parameter zUh also being described in the book The relation between surface tension and solubility parameter in liquids, Bagda, E, Farbe Lack, 84, 212, 1978; and V is the volume of the molecule.

It should be noted that the value of the solubility parameter δH is usually given for a temperature of 25°C and at atmospheric pressure (760 mmHg, i.e. $1.013 \times 10^5$ Pa).

In particular, the liquid organic compounds with a Hansen solubility parameter δH value of greater than 0 and less than 16 MPa1/2 are nonionic compounds.

Preferably, said liquid organic solvent(s) are chosen from alcohol ethers, aliphatic esters, aliphatic ethers, aromatic ethers, alkanols bearing aryl substituents, lactones and mixtures thereof.

Said liquid organic solvent(s) may preferably be chosen from:

- alcohol ethers, in particular C1-C4 ethers of C5-C30 alcohols, which are preferably saturated, linear or branched, optionally interrupted with one or more non-adjacent ether functions;

- aliphatic esters of C1-C4 carboxylic acids and of C3-C10 monoalcohols or polyhydroxylated alcohols, interrupted with one or more non-adjacent ether functions;

- aromatic ethers, in particular C6-C10 aromatic ethers, of a C1-C6 alkyl optionally bearing a hydroxyl group;

- (C6-C10)aryl(C1-C6)alkyl ethers, of a C1-C6 alkyl optionally bearing a hydroxyl group;

- alkanols bearing an aryl substituent, preferably for which the aryl part is a C6-C10 aryl part, advantageously a C6 aryl part, and the alkyl part of the alkanol is a C1-C4 alkyl part, this alkyl part possibly ending or being interrupted with a heteroatom, advantageously oxygen or a hydroxyl group, preferably such as benzyl alcohol;

- lactones preferably of formula (iii), and also mixtures thereof, with:

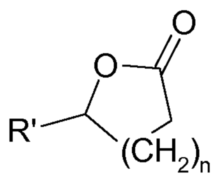

(iii)

in which R' represents a hydrogen, a linear or branched C1-C8 alkyl, a linear or branched C1-C4 hydroxyalkyl, n is equal to 1, 2 or 3, and preferably R' represents a hydrogen, a linear or branched C1-C6 alkyl or a linear or branched C1-C2 hydroxyalkyl.

A particularly advantageous example of lactones that may be mentioned is γ-butyrolactone.

Mention may also be made of certain liquid alkanols, for instance 1-pentanol.

Even more preferentially, said liquid organic solvent(s) are chosen from dipropylene glycol monomethyl ether acetate, dipropylene glycol methyl ether, dipropylene glycol mono(n-butyl) ether (the INCI name of which is PPG-2 Butyl Ether), tripropylene glycol methyl ether, propylene glycol n-butyl ether, propylene glycol n-propyl ether, propylene glycol monomethyl ether, diethylene glycol monomethyl ether and monoethyl ether, 3-phenyl-1-propanol, 2-phenyl-1-propanol, benzyl alcohol, benzyloxyethanol, phenoxyethanol, and mixtures of these compounds.

The liquid organic solvent(s) are preferably chosen from propylene glycol derivatives and aromatic alcohols, and mixtures thereof; even more preferentially chosen from alkanols bearing aryl substituents and even more preferentially benzyl alcohol and/or propylene glycol n-butyl ether.

Use may be made of other organic solvents, different from the liquid organic compound(s) with a Hansen solubility parameter δH value of greater than 0 and less than 16 $MPa^{1/2}$. Examples that may be mentioned include $C_1$-$C_4$ lower alkanols such as ethanol and isopropanol; polyols and polyol ethers.

Preferably, when they are present, the liquid organic solvent(s) represent a total content ranging from 0.1% to

35% by weight, preferably from 0.1% to 20% by weight and better still from 0.5% to 10% by weight, relative to the total weight of the composition.

*Direct dyes*

The composition according to the invention may optionally comprise b) one or more synthetic or natural direct dyes, chosen from cationic, anionic and nonionic species, preferably cationic or nonionic species.

Examples of suitable direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and natural direct dyes, alone or in the form of mixtures.

The direct dyes are preferably cationic direct dyes. Mention may be made of the hydrazono cationic dyes of formulae (IIIa) and (III'a), the azo cationic dyes (IVa) and (IV'a) and the diazo cationic dyes (Va) below:

$Het^+-C(R^a)=N-N(k^b)-Ar, An^-$ (IIIa)

$Het^+-N(R^a)-N=C(R^b)-Ar, An^-$ (III'a)

$Het^+-N=N-Ar, An^-$ (IVa)

$Ar^+-N=N-Ar'', An^-$ (IV'a)

and

$Het^+-N=N-Ar'-N=N-Ar, An^-$ (Va)

in which formulae (IIIa), (III'a), (IVa), (IV'a) and (Va):

- $Het^+$ represents a cationic heteroaryl radical, preferably bearing an endocyclic cationic charge, such as imidazolium, indolium or pyridinium, optionally substituted preferably with one or more $(C_1-C_8)$alkyl groups such as methyl;
- $Ar^+$ represents an aryl radical, such as phenyl or naphthyl, bearing an exocyclic cationic charge, preferably ammonium, particularly tri$(C_1-C_8)$alkylammonium such as trimethylammonium;
- Ar represents an aryl group, especially phenyl, which is optionally substituted, preferably with one or more electron-donating groups such as i) optionally substituted $(C_1-C_8)$alkyl, ii) optionally substituted $(C_1-C_8)$alkoxy, iii) (di)$(C_1-C_8)$(alkyl)amino optionally substituted on the alkyl group(s) with a hydroxyl group, iv) aryl$(C_1-C_8)$alkylamino, v) optionally substituted *N*-$(C_1-C_8)$alkyl-*N*-aryl$(C_1-C_8)$alkylamino or, as a variant, Ar represents a julolidine group;

- Ar' represents an optionally substituted divalent (hetero)arylene group such as phenylene, particularly para-phenylene, or naphthalene, which are optionally substituted, preferably with one or more groups $(C_1-C_8)$alkyl, hydroxyl or $(C_1-C_8)$alkoxy;
- Ar" represents an optionally substituted (hetero)aryl group such as phenyl or pyrazolyl, which are optionally substituted, preferably with one or more $(C_1-C_8)$alkyl, hydroxyl, (di)$(C_1-C_8)$(alkyl)amino, $(C_1-C_8)$alkoxy or phenyl groups;
- $R^a$ and $R^b$, which may be identical or different, represent a hydrogen atom or a $(C_1-C_8)$alkyl group, which is optionally substituted, preferably with a hydroxyl group;

  or, as a variant, the substituent $R^a$ with a substituent of $Het^+$ and/or $R^b$ with a substituent of Ar and/or $R^a$ with $R^b$ form, together with the atoms that bear them, a (hetero)cycloalkyl;

  particularly, $R^a$ and $R^b$ represent a hydrogen atom or a $(C_1-C_4)$alkyl group, which is optionally substituted with a hydroxyl group;

- $An^-$ represents an anionic counterion, such as mesylate or halide.

Mention may be made in particular of azo and hydrazono cationic dyes bearing an endocyclic cationic charge of formulae (IIIa), (III'a) and (IVa) as defined previously, more particularly those of formulae (IIIa), (III'a) and (IVa) derived from the dyes described in patent applications WO 95/15144, WO 95/01772 and EP-714954.

Preferably, the cationic part is derived from the following derivatives:

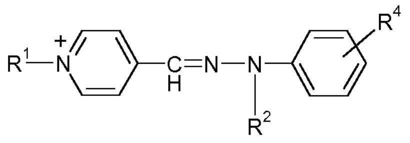

, $An^-$

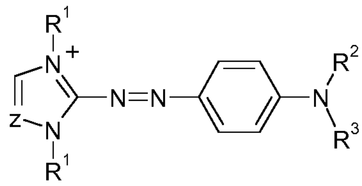

, $An^-$

(IIIa-1) (IVa-1)

formulae (III-1) and (IV-1) with:

- $R^1$ representing a $(C_1\text{-}C_4)$alkyl group such as methyl;
- $R^2$ and $R^3$, which may be identical or different, representing a hydrogen atom or a $(C_1\text{-}C_4)$alkyl group, such as methyl; and
- $R^4$ representing a hydrogen atom or an electron-donating group such as an optionally substituted $(C_1\text{-}C_8)$alkyl group, an optionally substituted $(C_1\text{-}C_8)$alkoxy group, or a (di)$(C_1\text{-}C_8)$(alkyl)amino group optionally substituted on the alkyl group(s) with a hydroxyl group; in particular, $R^4$ represents a hydrogen atom;
- Z representing a CH group or a nitrogen atom, preferably CH,
- $An^-$ representing an anionic counterion, such as mesylate or halide.

In particular, the dye of formulae (IIIa-1) and (IVa-1) is chosen from Basic Red 51, Basic Yellow 87 and Basic Orange 31 or corresponding derivatives:

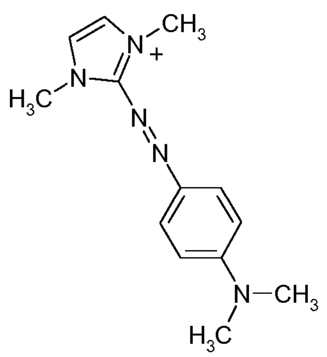

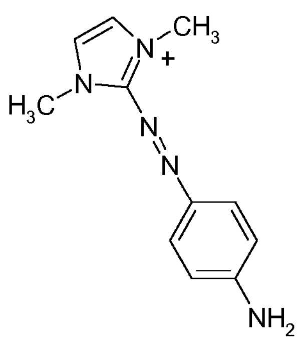

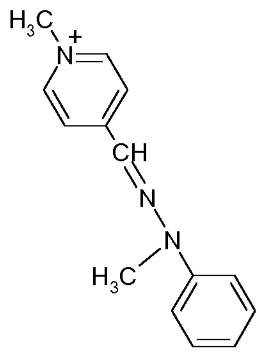

$An^-$ $An^-$ $An^-$

Among the natural direct dyes that may be used according to the invention, mention may be made of hennotannic acid, juglone, alizarin, purpurin, carminic acid, kermesic acid, purpurogallin, protocatechaldehyde, indigo, isatin, curcumin, spinulosin, apigenidin and orcein. Extracts or decoctions containing these natural dyes and in particular henna-based extracts or poultices may also be used.

When they are present, the direct dye(s) more particularly represent from 0.001% to 10% by weight and preferably from 0.005% to 5% by weight relative to the total weight of the composition.

According to a particular embodiment of the invention, the process is a dyeing process and the composition contains at least one direct dye as defined previously.

***Medium***

The cosmetically acceptable medium that is suitable for lightening keratin fibres, also known as the support, generally comprises water or a mixture of water and of at least one organic solvent as described previously or a mixture of organic solvents, to dissolve the compounds that would not be sufficiently water-soluble.

The compositions used according to the invention generally comprise water or a mixture of water and of one or

more organic solvents or a mixture of organic solvents.

The composition according to the invention preferably comprises water. Preferably, the water content ranges from 5% to 90% by weight, more preferentially from 10% to 80% by weight and better still from 20% to 70% by weight relative to the total weight of the composition.

***Cationic polymers***

According to an advantageous embodiment of the invention, the composition comprises one or more cationic polymers.

As cationic polymers that may be used in the compositions according to the invention, mention may be made in particular of:

(1) cyclopolymers of alkyldiallylamine or of dialkyldiallylammonium, such as the homopolymers or copolymers containing, as main constituent of the chain, units corresponding to formula (I) or (II):

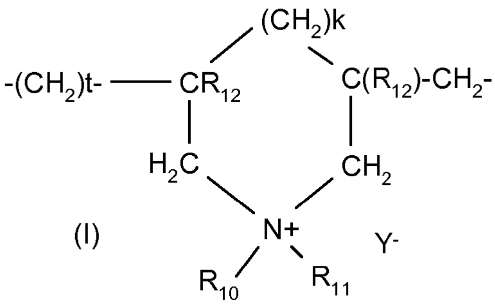
(I)

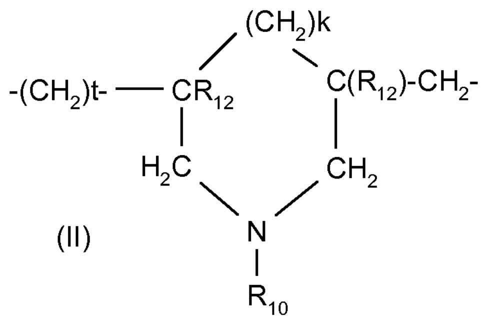
(II)

in which

- k and t are equal to 0 or 1, the sum k + t being equal to 1;
- $R_{12}$ denotes a hydrogen atom or a methyl radical;
- $R_{10}$ and $R_{11}$, independently of each other, denote a $C_1$-$C_6$ alkyl group, a $C_1$-$C_8$ hydroxyalkyl group, a $C_1$-$C_4$ amidoalkyl group; or alternatively $R_{10}$ and $R_{11}$ may denote, together with the nitrogen atom to which they are attached, a heterocyclic group such as piperidyl or morpholinyl; $R_{10}$ and $R_{11}$, independently of each other, preferably denote a $C_1$-$C_4$ alkyl group;
- $Y^-$ is an anion such as bromide, chloride, acetate, borate, citrate, tartrate, bisulfate, bisulfite, sulfate or phosphate.

Mention may be made more particularly of the dimethyldiallylammonium salt (for example chloride) homopolymer for example sold under the name Merquat 100 by the company Nalco. Preferably, the polymers of family (1) are chosen from dialkyldiallylammonium homopolymers.

(2) quaternary diammonium polymers comprising repeating units of formula:

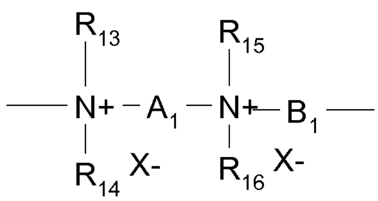
(III)

in which:

- $R_{13}$, $R_{14}$, $R_{15}$ and $R_{16}$, which may be identical or different, represent aliphatic, alicyclic or arylaliphatic radicals comprising from 1 to 20 carbon atoms or $C_1$-$C_{12}$ hydroxyalkyl aliphatic radicals,

  or else $R_{13}$, $R_{14}$, $R_{15}$ and $R_{16}$, together or separately, form, with the nitrogen atoms to which they are attached, heterocycles optionally comprising a second non-nitrogen heteroatom;
  or else $R_{13}$, $R_{14}$, $R_{15}$ and $R_{16}$ represent a linear or branched $C_1$-$C_6$ alkyl radical substituted with a nitrile, ester, acyl, amide or -CO-O-$R_{17}$-D or -CO-NH-$R_{17}$-D group, where $R_{17}$ is an alkylene and D is a quaternary

ammonium group;

- $A_1$ and $B_1$ represent linear or branched, saturated or unsaturated, divalent polymethylene groups comprising from 2 to 20 carbon atoms, which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
- $X^-$ denotes an anion derived from a mineral or organic acid;

it being understood that $A_1$, $R_{13}$ and $R_{15}$ can form, with the two nitrogen atoms to which they are attached, a piperazine ring;
in addition, if $A_1$ denotes a linear or branched, saturated or unsaturated alkylene or hydroxyalkylene radical, $B_1$ may also denote a group $(CH_2)n-CO-D-OC-(CH_2)p-$ with n and p, which may be identical or different, being integers ranging from 2 to 20, and D denoting:

a) a glycol residue of formula -O-Z-O-, in which Z denotes a linear or branched hydrocarbon-based radical, or a group corresponding to one of the following formulae: $-(CH_2CH_2O)x-CH_2CH_2-$ and $-[CH_2CH(CH_3)O]y-CH_2CH(CH_3)-$, in which x and y denote an integer from 1 to 4, representing a defined and unique degree of polymerization or any number from 1 to 4 representing an average degree of polymerization;
b) a bis-secondary diamine residue, such as a piperazine derivative;
c) a bis-primary diamine residue of formula -NH-Y-NH-, in which Y denotes a linear or branched hydrocarbon-based radical, or else the divalent radical $-CH_2-CH_2-S-S-CH_2-CH_2-$;
d) a ureylene group of formula -NH-CO-NH-.

Preferably, $X^-$ is an anion, such as chloride or bromide. These polymers have a number-average molar mass (Mn) generally of between 1000 and 100 000.

Mention may be made more particularly of cationic polymers that are constituted of repeating units corresponding to the formula:

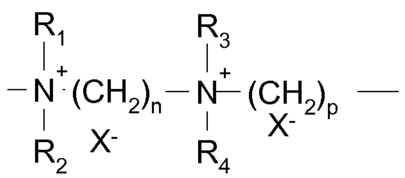

(IV)

in which $R_1$, $R_2$, $R_3$ and $R_4$, which may be identical or different, denote an alkyl or hydroxyalkyl radical containing from 1 to 4 carbon atoms, n and p are integers ranging from 2 to 20, and $X^-$ is an anion derived from a mineral or organic acid.
A particularly preferred compound of formula (IV) is the one for which $R_1$, $R_2$, $R_3$ and $R_4$ represent a methyl radical and n = 3, p = 6 and X = Cl, known as Hexadimethrine chloride according to the INCI (CTFA) nomenclature.

Preferably, the cationic polymer(s) are chosen from dialkyldiallylammonium homopolymers, in particular homopolymers of dimethyldiallylammonium salts, polymers constituted of repeating units corresponding to formula (IV) above, in particular poly(dimethyliminio)-1,3-propanediyl(dimethyliminio)-1,6-hexanediyl dichloride, the INCI name of which is hexadimethrine chloride, and mixtures thereof.

When they are present, the concentration of cationic polymers in the composition according to the present invention may range from 0.01% to 10% by weight relative to the weight of the composition, preferably from 0.1% to 5% relative to the weight of the composition, and even more advantageously from 0.2% to 3% by weight relative to the weight of the composition.

***Other additives***

The composition according to the invention may also contain various adjuvants conventionally used in hair lightening compositions, such as anionic, nonionic, amphoteric or zwitterionic polymers or mixtures thereof; mineral thickeners, and in particular fillers such as clays or talc; organic thickeners with, in particular, anionic, cationic, nonionic and amphoteric polymeric associative thickeners; antioxidants; penetrants; sequestrants; fragrances; dispersants; film-forming agents; ceramides; preserving agents; opacifiers.

The above adjuvants are generally present in an amount for each of them of between 0.01% and 40% by weight relative to the weight of the composition, and preferably between 0.1% and 20% by weight relative to the weight of the

composition.

Needless to say, a person skilled in the art will take care to select this or these possible additional compound(s) such that the advantageous properties intrinsically associated with the composition(s) that are useful in the lightening process in accordance with the invention are not, or are not substantially, adversely affected by the envisaged addition(s).

The composition may especially comprise one or more thickeners. In particular, the thickeners may be mineral thickeners chosen from organophilic clays and fumed silicas, or mixtures thereof.

The organophilic clay may be chosen from montmorillonite, bentonite, hectorite, attapulgite and sepiolite, and mixtures thereof. The clay is preferably a bentonite or a hectorite.

These clays may be modified with a chemical compound chosen from quaternary amines, tertiary amines, amine acetates, imidazolines, amine soaps, fatty sulfates, alkylarylsulfonates and amine oxides, and mixtures thereof.

Mention may be made, as organophilic clays, of quaternium-18 bentonites, such as those sold under the names Bentone 3, Bentone 38 and Bentone 38V by Rheox, Tixogel VP by United Catalyst and Claytone 34, Claytone 40 and Claytone XL by Southern Clay; stearalkonium bentonites, such as those sold under the names Bentone 27 by Rheox, Tixogel LG by United Catalyst and Claytone AF and Claytone APA by Southern Clay; and quaternium-18/benzalkonium bentonites, such as those sold under the names Claytone HT and Claytone PS by Southern Clay.

The fumed silicas may be obtained by high-temperature hydrolysis of a volatile silicon compound in an oxyhydrogen flame, producing a finely divided silica. This process makes it possible in particular to obtain hydrophilic silicas bearing a large number of silanol groups at their surface. Such hydrophilic silicas are sold, for example, under the names Aerosil 130®, Aerosil 2000, Aerosil 255®, Aerosil 300® and Aerosil 380® by Degussa and Cab-O-Sil HS-5®, Cab-O-Sil EH-5®, Cab-O-Sil LM-130®, Cab-O-Sil MS-55® and Cab-O-Sil M-5® by Cabot.

It is possible to chemically modify the surface of the silica via chemical reaction in order to reduce the number of silanol groups. It is especially possible to substitute silanol groups with hydrophobic groups: a hydrophobic silica is then obtained.

The hydrophobic groups may be:

- trimethylsiloxyl groups, which are obtained in particular by treating fumed silica in the presence of hexamethyldisilazane. Silicas thus treated are known as Silica silylate according to the CTFA (6th Edition, 1995). They are sold, for example, under the references Aerosil R812® by Degussa and Cab-O-Sil TS-530® by Cabot.
- dimethylsilyloxyl or polydimethylsiloxane groups, which are obtained in particular by treating fumed silica in the presence of polydimethylsiloxane or dimethyldichlorosilane. Silicas thus treated are known as Silica dimethyl silylate according to the CTFA (6th Edition, 1995). They are sold, for example, under the references Aerosil R972® and Aerosil R974® by the company Degussa and Cab-O-Sil TS-610® and Cab-O-Sil TS-720® by the company Cabot.

The fumed silica preferably has a particle size that may be nanometric to micrometric, for example ranging from about 5 to 200 nm.

When it is present, the mineral thickener represents from 1% to 30% by weight relative to the weight of the composition.

The composition may also comprise one or more organic thickeners.

These thickeners may be chosen from fatty acid amides (coconut monoethanolamide or diethanolamide, oxyethylenated carboxylic acid alkyl ether monoethanolamide), polymeric thickeners such as cellulose-based thickeners (hydroxyethylcellulose, hydroxypropylcellulose or carboxymethylcellulose), guar gum and derivatives thereof (hydroxypropyl guar), gums of microbial origin (xanthan gum, scleroglucan gum), crosslinked acrylic acid or acrylamidopropanesulfonic acid homopolymers and associative polymers (polymers comprising hydrophilic regions and fatty-chain hydrophobic regions (alkyl or alkenyl containing at least 10 carbon atoms) that are capable, in an aqueous medium, of reversibly combining with each other or with other molecules).

According to a particular embodiment, the organic thickener is chosen from cellulose-based thickeners (hydroxyethylcellulose, hydroxypropylcellulose or carboxymethylcellulose), guar gum and derivatives thereof (hydroxypropyl guar), gums of microbial origin (xanthan gum or scleroglucan gum) and crosslinked acrylic acid or acrylamidopropanesulfonic acid homopolymers, and preferably from cellulose-based thickeners in particular with hydroxyethylcellulose.

The content of organic thickener(s), if they are present, usually ranges from 0.01% to 20% by weight relative to the weight of the composition, and preferably from 0.1% to 5% by weight.

The composition of the invention may be in various forms, for instance a solution, an emulsion (milk or cream) or a gel, preferably in the form of an emulsion and particularly of a direct emulsion.

Preferably, the lightening composition according to the invention comprises:

(a) hydrogen peroxide,
(b) one or more carbonates and/or one or more carbonate-generating systems,

(c) one or more polyphosphorus derivatives preferably chosen from linear or cyclic compounds comprising at least two phosphorus atoms covalently bonded together via at least one linker L comprising at least one oxygen atom and/or at least one carbon atom, preferably at least one carbon atom, in a total content of greater than or equal to 0.5% by weight, relative to the total weight of the composition,
(d) preferably, one or more surfactants,
(e) preferably, one or more fatty substances, preferably non-silicone fatty substances,
(f) preferably, one or more liquid organic solvents,

the pH of said composition preferably being less than or equal to 9.7, preferably less than or equal to 9.5;
the composition being free of persalts.

Preferably, the lightening composition according to the invention comprises:

(a) hydrogen peroxide,
(b) one or more carbonates and/or one or more carbonate-generating systems,
(c) one or more polyphosphorus derivatives preferably chosen from linear or cyclic compounds comprising at least two phosphorus atoms covalently bonded together via at least one linker L comprising at least one oxygen atom and/or at least one carbon atom, in a total content of greater than or equal to 0.5% by weight, relative to the total weight of the composition,
(d)preferably, one or more surfactants,
(e)preferably, one or more fatty substances in a content greater than or equal to 5%, preferably greater than or equal to 10% by weight, relative to the weight of the composition, and
(f) preferably, one or more liquid organic solvents,

the pH of said composition being less than or equal to 9.7, preferably less than or equal to 9.5;
the composition being free of persalts.

***Lightening process***

The lightening process according to the invention consists in applying the composition comprising at least ingredients (a) to (c) and optionally ingredients (d), and/or (e) and/or (f) as defined previously to keratin materials, preferably wet or dry keratin fibres. The composition is left in place for a time generally from 1 minute to 1 hour, preferably from 5 minutes to 30 minutes.
The temperature during the lightening process is conventionally between room temperature (between 15°C and 25°C) and 80°C and preferably between room temperature and 60°C.
On conclusion of the treatment, the keratin materials, preferably human keratin fibres, are optionally rinsed with water, optionally washed with a shampoo and then rinsed with water, before being dried or left to dry.
The composition according to the invention is preferably prepared by mixing at least two compositions. Preferably, the mixing of said at least two compositions is performed extemporaneously, before application of the composition according to the invention to the keratin fibres.
In a first variant of the invention, the composition according to the invention comprising at least the ingredients (a) to (c) and optionally ingredients (d), and/or (e) and/or (f) as defined previously results from the mixing of two compositions:

- a composition (A) comprising (a) hydrogen peroxide, and
- a composition (B) comprising (b) one or more carbonates and/or one or more carbonate-generating systems as defined previously, and (c) one or more polyphosphorus derivatives as defined previously,

such that the content of polyphosphorus derivatives (c) in the lightening composition according to the invention resulting from the mixing of compositions (A) + (B) is greater than or equal to 0.5% by weight relative to the total weight of the composition and such that the pH of the composition according to the invention resulting from the mixing of compositions (A) + (B) is less than or equal to 9.7, preferably less than or equal to 9.5 and such that the composition resulting from the mixing of compositions (A) and (B) is free of persalts.
Preferentially, at least one of the compositions (A) or (B) is aqueous.
Even more preferentially, both the compositions (A) and (B) are aqueous.
The term "aqueous composition" means a composition comprising at least 5% water. Preferably, an aqueous composition comprises more than 10% by weight of water and even more advantageously more than 20% by weight of water.
Preferably, composition (A) is aqueous.

***Device***

Finally, the invention relates to a multi-compartment device comprising at least a first compartment containing composition (A) as described above and at least a second compartment containing composition (B) as described above, the compositions of the compartments being intended to be mixed before application, to give the formulation after mixing according to the invention, on condition that the content of phosphate derivative(s) is greater than or equal to 0.5% by weight relative to the weight of the formulation derived from the mixing of the compositions of the various compartments, the pH of the formulation derived from the mixing of the compositions of the various compartments being less than or equal to 9.7, preferably less than or equal to 9.5, and that the composition resulting from the mixing of compositions (A) and (B) is free of persalts.

The examples that follow serve to illustrate the invention without, however, being limiting in nature.

In these examples, the colour of the locks was evaluated in the CIE L*a*b* system, using a Minolta Spectrophotometer CM2600D colorimeter.

In this L*a*b* system, the three parameters denote, respectively, the colour intensity (L*), the green/red colour axis (a*) and the blue/yellow colour axis (b*). The higher the value of L*, the lighter the colour. The higher the value of a*, the redder the colour and the higher the value of b*, the yellower the colour.

The variation or extent of the lightening between untreated locks of hair and locks of hair after treatment is defined by the parameter DE* and is calculated according to the following equation:

$$DE^* = \sqrt{(L^* - L_o^*)^2 + (a^* - a_o^*)^2 + (b^* - b_o^*)^2} \quad \text{(i)}$$

In this equation, the parameters L*, a* and b* represent the values measured on locks of hair after lightening and the parameters $L_0^*$, $a_0^*$ and $b_0^*$ represent the values measured on untreated locks of hair. The higher the DE* value, the better the lightening of the keratin fibres.

__Example 1:__

The following compositions were prepared from the following ingredients in the following proportions, indicated in grams:

| | Comp. A comparative | Comp. B invention | Comp. C invention | Comp. D invention | Comp. E invention |
|---|---|---|---|---|---|
| Sodium carbonate | 2 | 2 | 1.9 | 1.9 | 1.9 |
| Potassium pyrophosphate | | 6 | 5.6 | 5.6 | 5.6 |
| Water | 98 | 92 | 19.9 | 19.9 | 19.9 |
| Dodecane | | | 18.5 | 18.5 | 18.5 |
| Benzyl alcohol | | | 0.9 | 0.9 | 0.9 |
| 1-(2-Butoxy-1-methylethoxy)propan-2-ol | | | 9.3 | 9.3 | 9.3 |
| Sodium laureth sulfate at 26% in water | | | 44 | 44 | 44 |
| 1N hydrochloric acid | qs pH 10 | | | | |
| Pyrophosphoric acid | | qs pH 10 | - | qs pH 10.5 | qs pH 10.1 |
| pH | 10 | 10 | 11.3 | 10.5 | 10.1 |

87.5 g of each of the compositions A to E were mixed with 12.5 g of aqueous hydrogen peroxide solution (50% concentration) (20-volumes final).

The mixtures thus obtained were applied to locks of natural pigmented Caucasian hair with a tone depth of 4 (TD4).

The "mixture/lock" bath ratio is, respectively, 10/1 (g/g).

The leave-on time is 1 hour, on a hotplate set at 40°C. On conclusion of the leave-on time, the locks are rinsed and then dried under a drying hood at 40°C.

The colour of the locks was evaluated in the CIE L*a*b* system, using a Minolta CM3600D spectrocolorimeter.

| | Composition | pH applied composition | L* | a* | b* | DE* |
|---|---|---|---|---|---|---|
| Non-lightened pigmented natural reference hair TD4 | | | 19.12 | 2.81 | 3.54 | ----- |
| 87.5 g of composition with 12.5 g of aqueous hydrogen peroxide solution (at 50% concentration) (20-volumes final) | A | 9.2 | 26.48 | 7.18 | 11.04 | 11.18 |
| | B | 9.3 | 33.47 | 9.19 | 18.5 | 21.45 |
| | C | 9.6 | 40.42 | 9.14 | 22.75 | 29.38 |
| | D | 9.5 | 41.06 | 9.25 | 23.11 | 30.1 |
| | E | 9.3 | 39.07 | 9.05 | 21.79 | 27.75 |

Composition B according to the invention comprising a polyphosphorus compound makes it possible to obtain greater lightening of the keratin fibres than with comparative composition A (DE* 10 points higher and L greater with the composition according to the invention).

The examples of the invention lead to very pronounced levels of lightening and lie in a lightening of greater than 4 tones.

**Example 2:**

The following composition was prepared from the following ingredients in the following proportions, indicated in grams:

| | **Composition I** |
|---|---|
| Potassium carbonate | 2 |
| Potassium polytriphosphate | 3.9 |
| Water | 24.6 |
| Isododecane | 14.8 |
| Benzyl alcohol | 1 |
| 1-(2-Butoxy-1-methylethoxy)propan-2-ol | 7.9 |
| Sodium laureth sulfate at 26% in water | 44.3 |
| Pyrophosphoric acid | qs pH 10.1 |

87.5 g of composition I were mixed with 12.5 g of aqueous hydrogen peroxide solution (50% concentration) (20-volumes final). The compositions thus obtained were applied to locks of natural pigmented Caucasian hair with a tone depth of 4 (TD4).

The "mixture/lock" bath ratio is, respectively, 10/1 (g/g).

The leave-on time is 1 hour, on a hotplate set at 40°C. On conclusion of the leave-on time, the locks are rinsed and then dried under a drying hood at 40°C.

The colour of the locks was evaluated in the CIE L*a*b* system, using a Minolta CM3600D spectrocolorimeter.

| | pH applied composition | L* | a* | b* | DE* |
|---|---|---|---|---|---|
| Non-lightened pigmented natural reference hair TD4 | | 19.34 | 2.91 | 3.77 | --- |
| 87.5 g of composition I with 12.5 g of aqueous hydrogen peroxide solution (at 50% concentration) (20-volumes final) | 8.7 | 31.06 | 8.85 | 16.26 | 18.31 |

The compositions according to the invention give very pronounced levels of lightening.

**Claims**

1. Composition for lightening keratin materials, preferably keratin fibres, in particular human keratin fibres such as the hair, comprising:

   (a) hydrogen peroxide;
   (b) one or more carbonates and/or one or more carbonate-generating systems;
   (c) one or more polyphosphorus derivatives preferably chosen from linear or cyclic compounds comprising at least two phosphorus atoms covalently bonded together via at least one linker L comprising at least one oxygen atom and/or at least one carbon atom, preferably at least one oxygen atom, in a total content of greater than or equal to 0.5% by weight, relative to the total weight of the composition;

   and the pH of the composition is less than or equal to 9.7, the composition being free of persalts.

2. Composition according to Claim 1, **characterized in that** hydrogen peroxide represents from 0.1% to 25% by weight, preferably from 1% to 20% by weight or else more preferentially from 2% to 15% by weight, relative to the total weight of the composition.

3. Composition according to either of the preceding claims, **characterized in that** it is free of generators of peroxygenated salt(s).

4. Composition according to any one of the preceding claims, **characterized in that** the carbonate(s) and/or the carbonate-generating system(s) are chosen from carbonates of alkali metals ($Met_2^+$, $CO_3^{2-}$), of alkaline-earth metals ($Met'^{2+}$, $CO_3^{2-}$) or of phosphonium $(R''_4P^+)_2$,$CO_3^{2-}$ with Met' representing an alkaline-earth metal and Met representing an alkali metal, and R'', which may be identical or different, representing a hydrogen atom or an optionally substituted $(C_1$-$C_6)$alkyl group such as hydroxyethyl; and mixtures thereof.

5. Composition according to any one of the preceding claims, **characterized in that** the carbonate(s) and/or the carbonate-generating system(s) are chosen from alkali metal or alkaline-earth metal carbonates, and mixtures thereof; preferentially alkali metal carbonates, and mixtures thereof.

6. Composition according to any one of the preceding claims, **characterized in that** the carbonate(s) and/or the carbonate-generating system(s) are chosen from Na, K, Mg, Ca carbonates and mixtures thereof, preferably from sodium and/or potassium bicarbonate.

7. Composition according to any one of the preceding claims, **characterized in that** the carbonate(s) represent from 0.01% to 20% by weight, relative to the total weight of the lightening composition, and even more preferentially from 0.1% to 15% and better still from 0.5% to 10% by weight, relative to the total weight of the composition.

8. Composition according to any one of the preceding claims, **characterized in that** said polyphosphorus derivative(s) are chosen from linear or cyclic compounds comprising at least two phosphorus atoms covalently bonded together via at least one linker L comprising at least one oxygen atom and/or at least one carbon atom, preferably at least one oxygen atom.

9. Composition according to any one of the preceding claims, **characterized in that** said polyphosphorus derivative(s) are chosen from linear or cyclic compounds comprising at least two phosphorus atoms covalently bonded together via at least one linker L comprising at least one oxygen atom.

10. Composition according to any one of the preceding claims, **characterized in that** said polyphosphorus derivative(s) comprise at least two groups chosen from a group -P(R)(=O)-OH, a group -P(R)(=O)-O M, a group >P(=O)-OH and/or a group >P(=O)-O M, with:

   • M representing a cationic counterion, preferably chosen from alkali metals and alkaline-earth metals,
   • R representing a hydroxyl group, a group $-O^-$ M, with M representing a cationic counterion, preferably chosen from alkali metals and alkaline-earth metals, a $(C_1$-$C_6)$alkyl, $(C_1$-$C_6)$alkoxy, cycloalkyloxy or (hetero)aryloxy group, and
   > representing the two bonds connected to the phosphorus atom and forming part of a ring.

**11.** Composition according to any one of the preceding claims, **characterized in that** said polyphosphorus derivative(s) are chosen from compounds belonging to any one of formulae (I), (II) and (III) below; or mixtures thereof:

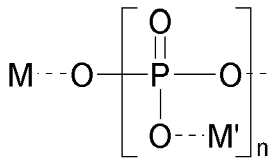
(I)

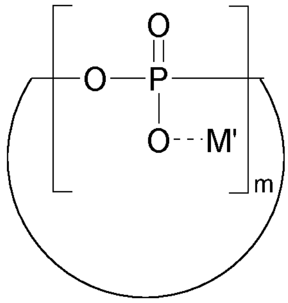
(II)

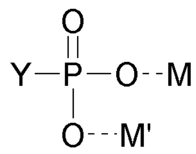
(III)

and also the solvates thereof such as hydrates;
with

- n ranging from 2 to 10, preferably from 2 to 6 and better still from 2 to 3;
- m ranging from 2 to 10, preferably from 2 to 6;
Y representing an alkyl chain comprising at least one phosphorus atom and optionally one or more non-phosphorus heteroatoms, or a cyclic carbon-based radical optionally comprising one or more heteroatoms, said hydrocarbon-based radical being substituted with one or more groups comprising one or more phosphorus atoms;
- M or M' representing a hydrogen atom, an alkali metal or an alkaline-earth metal;
- - - - - representing a single bond when M or M' is H, or an ionic bond.

**12.** Composition according to any one of the preceding claims, **characterized in that** the polyphosphorus derivative(s) are chosen from:

- inorganic polyphosphorus derivatives chosen from:

o pyrophosphates, preferably in the form of salts, preferably of alkali metal salts, which may or may not be hydrated, such as sodium pyrophosphate, potassium pyrophosphate or sodium pyrophosphate decahydrate;
o hexametaphosphates, preferably in the form of salts, preferably of alkali metal salts, which may or may not be hydrated, such as sodium hexametaphosphate;
o tripolyphosphates, preferably in the form of salts, preferably of alkali metal salts, which may or may not be hydrated, such as sodium tripolyphosphate;
o trimetaphosphates, preferably in the form of salts, preferably of alkali metal salts, which may or may not be hydrated, such as sodium trimetaphosphate;
◦ and mixtures thereof;

- and organic polyphosphorus derivatives, preferably chosen from:

o organic polyphosphate derivatives, such as polyphosphoric acids and/or salts thereof such as phytic acid;
o organic polyphosphonate derivatives, such as polyphosphonic acids and/or salts thereof such as EDTMP, DETMP, ATMP, HEDP, DTPMP, and mixtures thereof;

- and mixtures thereof.

**13.** Composition according to any one of the preceding claims, **characterized in that** the polyphosphorus derivative(s) are chosen from:

- inorganic polyphosphate derivatives chosen from hydrated or non-hydrated alkali metal pyrophosphates, such as sodium pyrophosphate, potassium pyrophosphate, sodium pyrophosphate decahydrate; and polyphosphates, such as sodium hexametaphosphate, sodium polyphosphate, sodium tripolyphosphate, sodium

trimetaphosphate; and mixtures thereof;
- organic polyphosphorus derivatives chosen from polyphosphoric acids and/or salts thereof such as phytic acid, polyphosphonic acids and/or salts thereof such as EDTMP, DETMP, ATMP, HEDP, DTPMP, and mixtures thereof;
- and mixtures thereof;

preferably from inorganic polyphosphate derivatives.

**14.** Composition according to any one of the preceding claims, **characterized in that** the polyphosphorus derivative(s) are chosen from inorganic polyphosphate derivatives, preferably from hydrated or non-hydrated alkali metal pyrophosphates, preferably from sodium pyrophosphate, potassium pyrophosphate and sodium pyrophosphate decahydrate, and mixtures thereof.

**15.** Composition according to any one of the preceding claims, **characterized in that** the polyphosphorus derivative(s) represent from 0.5% to 20% by weight, relative to the weight of the lightening composition containing them, preferably from 0.55% to 15% by weight, better still from 0.7% to 12% by weight.

**16.** Composition according to any one of the preceding claims, **characterized in that** the polyphosphorus derivative(s) are present in a total content ranging from 1% to 10% by weight relative to the total weight of the composition.

**17.** Composition according to any one of the preceding claims, **characterized in that** the polyphosphorus derivative(s) are present in a total content ranging from 2% to 10% by weight and preferably from 2.5% to 10% by weight relative to the total weight of the composition.

**18.** Composition according to any one of the preceding claims, **characterized in that** it comprises one or more surfactants, preferably chosen from anionic and/or nonionic surfactants, preferably nonionic surfactants.

**19.** Composition according to any one of the preceding claims, **characterized in that** it comprises one or more fatty substances, preferably non-silicone fatty substances, preferably chosen from hydrocarbons with more than 16 carbon atoms, $C_6$-$C_{16}$ alkanes, oils or triglycerides of plant origin, liquid synthetic triglycerides, fatty alcohols, esters of a fatty acid and/or of a fatty alcohol other than triglycerides, and non-silicone waxes, or mixtures thereof, preferably chosen from hydrocarbons with more than 16 carbon atoms, such as liquid petroleum jelly, liquid paraffin and polydecenes; liquid fatty alcohols such as octyldodecanol, fatty acids, liquid esters of fatty acids and/or of fatty alcohols, or mixtures thereof.

**20.** Composition according to the preceding claim, **characterized in that** the fatty substance(s) represent a total content ranging from 5% to 80% by weight, more preferentially from 10% to 80% by weight, preferably from 15% to 75% by weight, better still from 20% to 70% by weight, even more advantageously from 25% to 70% and preferentially from 25% to 60% by weight relative to the total weight of the composition.

**21.** Composition according to any one of the preceding claims, **characterized in that** it comprises one or more liquid organic solvents, preferably with a Hansen solubility parameter $\delta H$ value of greater than 0 and less than 16 MPa1/2, preferably chosen from alcohol ethers, aliphatic esters, aliphatic ethers, aromatic ethers, alkanols bearing aryl substituents, lactones, and mixtures thereof, preferably chosen from propylene glycol derivatives and aromatic alcohols, and mixtures thereof, preferably benzyl alcohol and/or propylene glycol n-butyl ether, preferably in a total content ranging from 0.1% to 35% by weight, preferably from 0.1% to 20% by weight and better still from 0.5% to 10% by weight relative to the total weight of the composition.

**22.** Composition according to any one of the preceding claims, **characterized in that** the pH ranges from 7 to 9.7, preferably the pH ranges between 7 and 8.7.

**23.** Composition according to any one of the preceding claims, **characterized in that** it comprises at least a basifying agent d), preferably chosen from i) alkanolamines such as monoethanolamine, diethanolamine, triethanolamine and derivatives thereof, ii) oxyethylenated and/or oxypropylenated ethylenediamines, iii) mineral or organic hydroxides, iv) alkali metal silicates such as sodium metasilicates, v) amino acids; and mixture thereof.

**24.** Process for lightening keratin materials, in particular keratin fibres, preferably human keratin fibres such as the hair, in which the composition as defined according to any one of Claims 1 to 23 is applied to said keratin materials, preferably keratin fibres.

**25.** Lightening process according to the preceding claim, **characterized in that** the composition as defined according to any one of Claims 1 to 23 is derived from the mixing of at least two compositions, and in particular of two compositions:

- a composition (A) comprising (a) hydrogen peroxide, and
- a composition (B) comprising one or more carbonates and/or one or more carbonate-generating systems, and (c) one or more polyphosphorus derivatives as defined according to any one of Claims 1 to 23,

such that the content of c) polyphosphorus derivative(s) of the composition resulting from the mixing of compositions (A) + (B) is greater than or equal to 0.5% by weight relative to the total weight of the composition and such that the pH of the composition resulting from the mixing of compositions (A) + (B) is less than or equal to 9.7, preferably less than or equal to 9.5, and such that the composition resulting from the mixing of compositions (A) and (B) is free of persalts.

**26.** Multi-compartment device comprising at least a first compartment containing the composition (A) as defined in Claim 25 and at least a second compartment containing the composition (B) as defined in Claim 25, the compositions of the compartments being intended to be mixed before application, to give the formulation after mixing, to give the formulation after mixing, on condition that the content of c) polyphosphorus derivative(s), as defined according to any one of Claims 1 to 22, of the composition resulting from the mixing of compositions (A) + (B) is greater than or equal to 0.5% by weight relative to the weight of the formulation derived from the mixing of (A)+(B) and that the pH of the formulation resulting from the mixing of (A)+(B) is less than or equal to 9.7, preferably less than or equal to 9.5, and that the composition resulting from the mixing of compositions (A) and (B) is free of persalts.

## Patentansprüche

**1.** Zusammensetzung zum Aufhellen von Keratinmaterialien, vorzugsweise Keratinfasern, insbesondere menschlichen Keratinfasern wie dem Haar, umfassend:

(a) Wasserstoffperoxid;
(b) ein oder mehrere Carbonate und/oder ein oder mehrere carbonaterzeugende Systeme;
(c) ein oder mehrere Polyphosphorderivate, vorzugsweise ausgewählt aus linearen oder cyclischen Verbindungen mit mindestens zwei Phosphoratomen, die über mindestens einen Linker L mit mindestens einem Sauerstoffatom und/oder mindestens einem Kohlenstoffatom, vorzugsweise mindestens einem Sauerstoffatom, kovalent aneinander gebunden sind, in einem Gesamtgehalt größer oder gleich 0,5 Gew.-%, bezogen auf das Gesamtgewicht der Zusammensetzung;

und der pH-Wert der Zusammensetzung kleiner oder gleich 9,7 ist, wobei die Zusammensetzung frei von Persalzen ist.

**2.** Zusammensetzung nach Anspruch 1, **dadurch gekennzeichnet, dass** Wasserstoffperoxid 0,1 bis 25 Gew.- %, vorzugsweise 1 bis 20 Gew.-% oder auch weiter bevorzugt 2 bis 15 Gew.-%, bezogen auf das Gesamtgewicht der Zusammensetzung, ausmacht.

**3.** Zusammensetzung nach einem der vorhergehenden Ansprüche, **dadurch gekennzeichnet, dass** sie frei von Erzeugern von peroxygeniertem Salz bzw. peroxygenierten Salzen ist.

**4.** Zusammensetzung nach einem der vorhergehenden Ansprüche, **dadurch gekennzeichnet, dass** das Carbonat bzw. die Carbonate und/oder das carbonaterzeugende System bzw. die carbonaterzeugenden Systeme aus Carbonaten von Alkalimetallen ($Met_2^+$, $CO_3^{2-}$), von Erdalkalimetallen ($Met'^{2+}$, $CO_3^{2-}$) oder von Phosphonium $(R''_4P^+)_2,CO_3^{2-}$, wobei Met' für ein Erdalkalimetall steht und Met für ein Alkalimetall steht, und die Variablen R'', die gleich oder verschieden sein können, für ein Wasserstoffatom oder eine gegebenenfalls substituierte ($C_1$-$C_6$)-Alkylgruppe wie Hydroxyethyl stehen; und Mischungen davon ausgewählt ist bzw. sind.

**5.** Zusammensetzung nach einem der vorhergehenden Ansprüche, **dadurch gekennzeichnet, dass** das Carbonat bzw. die Carbonate und/oder das carbonaterzeugende System bzw. die carbonaterzeugenden Systeme aus Alkali- oder Erdalkalimetallcarbonaten und Mischungen davon, bevorzugt Alkalimetallcarbonaten und Mischungen davon, ausgewählt ist bzw. sind.

**6.** Zusammensetzung nach einem der vorhergehenden Ansprüche, **dadurch gekennzeichnet, dass** das Carbonat

bzw. die Carbonate und/oder das carbonaterzeugende System bzw. die carbonaterzeugenden Systeme aus Na-, K-, Mg- und Ca-Carbonaten und Mischungen davon, vorzugsweise aus Natrium- und/oder Kaliumhydrogencarbonat, ausgewählt ist bzw. sind.

**7.** Zusammensetzung nach einem der vorhergehenden Ansprüche, **dadurch gekennzeichnet, dass** das Carbonat bzw. die Carbonate 0,01 bis 20 Gew.-%, bezogen auf das Gesamtgewicht der Aufhellungszusammensetzung, und noch weiter bevorzugt 0,1 bis 15 Gew.-% und noch besser 0,5 bis 10 Gew.-%, bezogen auf das Gesamtgewicht der Zusammensetzung, ausmacht bzw. ausmachen.

**8.** Zusammensetzung nach einem der vorhergehenden Ansprüche, **dadurch gekennzeichnet, dass** das Polyphosphorderivat bzw. die Polyphosphorderivate aus linearen oder cyclischen Verbindungen mit mindestens zwei Phosphoratomen, die über mindestens einen Linker L mit mindestens einem Sauerstoffatom und/oder mindestens einem Kohlenstoffatom, vorzugsweise mindestens einem Sauerstoffatom, kovalent aneinander gebunden sind, ausgewählt ist bzw. sind.

**9.** Zusammensetzung nach einem der vorhergehenden Ansprüche, **dadurch gekennzeichnet, dass** das Polyphosphorderivat bzw. die Polyphosphorderivate aus linearen oder cyclischen Verbindungen mit mindestens zwei Phosphoratomen, die über mindestens einen Linker L mit mindestens einem Sauerstoffatom kovalent aneinander gebunden sind, ausgewählt ist bzw. sind.

**10.** Zusammensetzung nach einem der vorhergehenden Ansprüche, **dadurch gekennzeichnet, dass** das Polyphosphorderivat bzw. die Polyphosphorderivate mindestens zwei Gruppen umfasst bzw. umfassen, die aus einer Gruppe -P(R) (=O)-OH, einer Gruppe -P(R) (=O)-O M, einer Gruppe >P(=O)-OH und/oder einer Gruppe >P(=O)-O M ausgewählt sind, wobei:

- M für ein kationisches Gegenion steht, das vorzugsweise aus Alkalimetallen und Erdalkalimetallen ausgewählt ist,
- R für eine Hydroxylgruppe, eine $-O^-$ M-Gruppe, wobei M für ein kationisches Gegenion steht, das vorzugsweise aus Alkali- und Erdalkalimetallen ausgewählt ist, eine $(C_1-C_6)$Alkyl-, $(C_1-C_6)$Alkoxy-, Cycloalkyloxy- oder (Hetero)aryloxygruppe steht und

> für die zwei Bindungen, die an das Phosphoratom gebunden sind und Teil eines Rings bilden, steht.

**11.** Zusammensetzung nach einem der vorhergehenden Ansprüche, **dadurch gekennzeichnet, dass** das Polyphosphorderivat bzw. die Polyphosphorderivate aus Verbindungen ausgewählt ist bzw. sind, die zu einer der folgenden Formeln (I), (II) und (III) gehören; oder Mischungen davon:

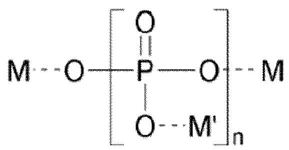

**(I)**

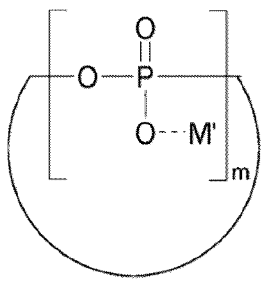

**(II)**

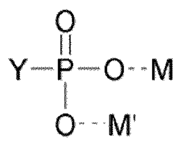

**(III)**

sowie den Solvaten davon wie Hydraten ausgewählt ist bzw. sind;
wobei

- n im Bereich von 2 bis 10, vorzugsweise von 2 bis 6 und noch besser von 2 bis 3 liegt;
- m im Bereich von 2 bis 10, vorzugsweise von 2 bis 6, liegt;

Y für eine Alkylkette mit mindestens einem Phosphoratom und gegebenenfalls einem oder mehreren Nicht-Phosphor-Heteroatomen oder einen cyclischen Rest auf Kohlenstoffbasis, der gegebenenfalls ein oder mehrere Heteroatome umfasst, steht, wobei der Rest auf Kohlenwasserstoffbasis durch eine oder mehrere Gruppen, die ein oder mehrere Phosphoratome umfassen, substituiert ist;

- M oder M’ für ein Wasserstoffatom, ein Alkalimetall oder ein Erdalkalimetall stehen;

- - - - - für eine Einfachbindung steht, wenn M oder M' für H steht, oder für eine ionische Bindung steht.

**12.** Zusammensetzung nach einem der vorhergehenden Ansprüche, **dadurch gekennzeichnet, dass** das Polyphosphorderivat bzw. die Polyphosphorderivate ausgewählt ist bzw. sind aus:

- anorganischen Polyphosphorderivaten, ausgewählt aus:

  o Pyrophosphaten, vorzugsweise in Form von Salzen, vorzugsweise von Alkalimetallsalzen, die gegebenenfalls hydratisiert sein können, wie Natriumpyrophosphat, Kaliumpyrophosphat oder Natriumpyrophosphatdecahydrat;
  o Hexametaphosphaten, vorzugsweise in Form von Salzen, vorzugsweise von Alkalimetallsalzen, die gegebenenfalls hydratisiert sein können, wie Natriumhexametaphosphat;
  o Tripolyphosphaten, vorzugsweise in Form von Salzen, vorzugsweise von Alkalimetallsalzen, die gegebenenfalls hydratisiert sein können, wie Natriumtripolyphosphat;
  o Trimetaphosphaten, vorzugsweise in Form von Salzen, vorzugsweise von Alkalimetallsalzen, die gegebenenfalls hydratisiert sein können, wie Natriumtrimetaphosphat;
  o und Mischungen davon;

- und organischen Polyphosphorderivaten, vorzugsweise ausgewählt aus:

  o organischen Polyphosphatderivaten, wie Polyphosphorsäuren und/oder Salzen davon wie Phytinsäure;
  o organischen Polyphosphonatderivaten, wie Polyphosphonsäuren und/oder Salzen davon, wie EDTMP, DETMP, ATMP, HEDP, DTPMP und Mischungen davon;

- und Mischungen davon.

**13.** Zusammensetzung nach einem der vorhergehenden Ansprüche, **dadurch gekennzeichnet, dass** das Polyphosphorderivat bzw. die Polyphosphorderivate ausgewählt ist bzw. sind aus:

- anorganischen Polyphosphatderivaten, ausgewählt aus hydratisierten oder nicht hydratisierten Alkalimetallpyrophosphaten, wie Natriumpyrophosphat, Kaliumpyrophosphat, Natriumpyrophosphatdecahydrat; und Polyphosphaten, wie Natriumhexametaphosphat, Natriumpolyphosphat, Natriumtripolyphosphat, Natriumtrimetaphosphat; und Mischungen davon;
- organischen Polyphosphorderivaten, ausgewählt aus Polyphosphorsäuren und/oder Salzen davon wie Phytinsäure, Polyphosphonsäuren und/oder Salzen davon wie EDTMP, DETMP, ATMP, HEDP, DTPMP und Mischungen davon;
- und Mischungen davon;

vorzugsweise aus anorganischen Polyphosphatderivaten.

**14.** Zusammensetzung nach einem der vorhergehenden Ansprüche, **dadurch gekennzeichnet, dass** das Polyphosphorderivat **bzw.** die Polyphosphorderivate aus anorganischen Polyphosphatderivaten, vorzugsweise aus hydratisierten oder nicht hydratisierten Alkalimetallpyrophosphaten, vorzugsweise aus Natriumpyrophosphat, Kaliumpyrophosphat und Natriumpyrophosphatdecahydrat, und Mischungen davon ausgewählt ist bzw. sind.

**15.** Zusammensetzung nach einem der vorhergehenden Ansprüche, **dadurch gekennzeichnet, dass** das Polyphosphorderivat bzw. die Polyphosphorderivate 0,5 bis 20 Gew.-%, bezogen auf das Gewicht der sie enthaltenden Aufhellungszusammensetzung, vorzugsweise 0,55 bis 15 Gew.-%, noch besser 0,7 bis 12 Gew.-%, ausmacht bzw. ausmachen.

**16.** Zusammensetzung nach einem der vorhergehenden Ansprüche, **dadurch gekennzeichnet, dass** das Polyphosphorderivat bzw. die Polyphosphorderivate in einem Gesamtgehalt im Bereich von 1 Gew.-% bis 10 Gew.-%, bezogen auf das Gesamtgewicht der Zusammensetzung, vorliegt bzw. vorliegen.

**17.** Zusammensetzung nach einem der vorhergehenden Ansprüche, **dadurch gekennzeichnet, dass** das Polyphosphorderivat bzw. die Polyphosphorderivate in einem Gesamtgehalt im Bereich von 2 bis 10 Gew.-% und vorzugsweise von 2,5 bis 10 Gew.-%, bezogen auf das Gesamtgewicht der Zusammensetzung, vorliegt bzw. vorliegen.

**18.** Zusammensetzung nach einem der vorhergehenden Ansprüche, **dadurch gekennzeichnet, dass** sie ein oder mehrere Tenside umfasst, die vorzugsweise aus anionischen und/oder nichtionischen Tensiden, vorzugsweise nichtionischen Tensiden, ausgewählt sind.

**19.** Zusammensetzung nach einem der vorhergehenden Ansprüche, **dadurch gekennzeichnet, dass** sie eine oder mehrere Fettsubstanzen, vorzugsweise Nicht-Silikon-Fettsubstanzen, vorzugsweise ausgewählt aus Kohlenwasserstoffen mit mehr als 16 Kohlenstoffatomen, $C_6$-$C_{16}$-Alkanen, Ölen oder Triglyceriden pflanzlicher Herkunft, flüssigen synthetischen Triglyceriden, Fettalkoholen, Estern einer Fettsäure und/oder eines Fettalkohols, die von Triglyceriden verschieden sind, und Nicht-Silikon-Wachsen oder Mischungen davon, vorzugsweise ausgewählt aus Kohlenwasserstoffen mit mehr als 16 Kohlenstoffatomen, wie Vaselineöl, Flüssigparaffin und Polydecenen; flüssigen Fettalkoholen wie Octyldodecanol, Fettsäuren, flüssigen Estern von Fettsäuren und/oder von Fettalkoholen oder Mischungen davon, umfasst.

**20.** Zusammensetzung nach dem vorhergehenden Anspruch, **dadurch gekennzeichnet, dass** die Fettsubstanz bzw. die Fettsubstanzen einen Gesamtgehalt im Bereich von 5 bis 80 Gew.-%, weiter bevorzugt von 10 bis 80 Gew.-%, vorzugsweise von 15 bis 75 Gew.-%, noch besser von 20 bis 70 Gew.-%, noch vorteilhafter von 25 bis 70 Gew.-% und bevorzugt von 25 bis 60 Gew.-%, bezogen auf das Gesamtgewicht der Zusammensetzung, ausmacht bzw. ausmachen.

**21.** Zusammensetzung nach einem der vorhergehenden Ansprüche, **dadurch gekennzeichnet, dass** sie ein oder mehrere flüssige organische Lösungsmittel, vorzugsweise mit einem Wert des Hansen-Löslichkeitsparameters $\delta H$ von mehr als 0 und weniger als 16 MPa1/2, vorzugsweise ausgewählt aus Alkoholethern, aliphatischen Estern, aliphatischen Ethern, aromatischen Ethern, Alkanolen mit Arylsubstituenten, Lactonen und Mischungen davon, vorzugsweise ausgewählt aus Propylenglykolderivaten und aromatischen Alkoholen und Mischungen davon, vorzugsweise Benzylalkohol und/oder Propylenglykol-n-butylether, umfasst, vorzugsweise in einem Gesamtgehalt im Bereich von 0,1 bis 35 Gew.-%, vorzugsweise von 0,1 bis 20 Gew.-% und noch besser von 0,5 bis 10 Gew.-%, bezogen auf das Gesamtgewicht der Zusammensetzung.

**22.** Zusammensetzung nach einem der vorhergehenden Ansprüche, **dadurch gekennzeichnet, dass** der pH-Wert im Bereich von 7 bis 9,7 liegt, vorzugsweise der pH-Wert zwischen 7 und 8,7 liegt.

**23.** Zusammensetzung nach einem der vorhergehenden Ansprüche, **dadurch gekennzeichnet, dass** sie mindestens ein Basifizierungsmittel d), vorzugsweise ausgewählt aus i) Alkanolaminen wie Monoethanolamin, Diethanolamin, Triethanolamin und Derivaten davon, ii) oxyethylenierten und/oder oxypropylenierten Ethylendiaminen, iii) mineralischen oder organischen Hydroxiden, iv) Alkalimetallsilikaten wie Natriummetasilikaten, v) Aminosäuren und Mischungen davon, umfasst.

**24.** Verfahren zum Aufhellen von Keratinmaterialien, insbesondere Keratinfasern, vorzugsweise menschlichen Keratinfasern wie dem Haar, bei dem die Zusammensetzung gemäß einem der Ansprüche 1 bis 23 auf die Keratinmaterialien, vorzugsweise Keratinfasern, aufgebracht wird.

**25.** Aufhellungsverfahren nach dem vorhergehenden Anspruch, **dadurch gekennzeichnet, dass** sich die Zusammensetzung gemäß einem der Ansprüche 1 bis 23 aus dem Mischen von mindestens zwei Zusammensetzungen und insbesondere von zwei Zusammensetzungen ergibt:

- einer Zusammensetzung (A), die (a) Wasserstoffperoxid umfasst, und
- einer Zusammensetzung (B), die ein oder mehrere Carbonate und/oder ein oder mehrere carbonaterzeugende Systeme und (c) ein oder mehrere Polyphosphorderivate gemäß einem der Ansprüche 1 bis 23 umfasst,

derart, dass der Gehalt der aus dem Mischen der Zusammensetzungen (A) + (B) resultierenden Zusammensetzung an c) Polyphosphorderivat(en) größer oder gleich 0,5 Gew.-%, bezogen auf das Gesamtgewicht der Zusammensetzung, ist, und derart, dass der pH-Wert der aus dem Mischen der Zusammensetzungen (A) + (B) resultierenden Zusammensetzung kleiner oder gleich 9,7, vorzugsweise kleiner oder gleich 9,5, ist, und derart, dass die aus dem Mischen der Zusammensetzungen (A) und (B) resultierende Zusammensetzung frei von Persalzen ist.

**26.** Vorrichtung mit mehreren Kompartimenten, umfassend mindestens ein erstes Kompartiment, das die Zusammensetzung (A) gemäß Anspruch 25 enthält, und mindestens ein zweites Kompartiment, das die Zusammensetzung (B) gemäß Anspruch 25 enthält, wobei die Zusammensetzungen der Kompartimente vor der Anwendung zu mischen

sind, was nach dem Mischen die Formulierung ergibt, mit der Maßgabe, dass der Gehalt der aus dem Mischen der Zusammensetzungen (A) + (B) resultierenden Zusammensetzung an c) Polyphosphorderivat(en) gemäß einem der Ansprüche 1 bis 22 größer oder gleich 0,5 Gew.- %, bezogen auf das Gewicht der aus dem Mischen von (A) + (B) resultierenden Formulierung, ist, und dass der pH-Wert der aus dem Mischen von (A) + (B) resultierenden Formulierung kleiner oder gleich 9,7, vorzugsweise kleiner oder gleich 9,5, ist und dass die aus dem Mischen der Zusammensetzungen (A) und (B) resultierende Zusammensetzung frei von Persalzen ist.

**Revendications**

1. Composition pour l'éclaircissement de matières kératiniques, de préférence de fibres kératiniques, en particulier de fibres kératiniques humaines telles que les cheveux, comprenant :

   (a) du peroxyde d'hydrogène ;
   (b) un ou plusieurs carbonates et/ou un ou plusieurs systèmes de génération de carbonate ;
   (c) un ou plusieurs dérivés polyphosphorés choisis de préférence parmi les composés linéaires ou cycliques comprenant au moins deux atomes de phosphore liés de manière covalente entre eux par l'intermédiaire d'au moins un lieur L comprenant au moins un atome d'oxygène et/ou au moins un atome de carbone, de préférence au moins un atome d'oxygène, en une teneur totale supérieure ou égale à 0,5 % en poids, par rapport au poids total de la composition ;

   et le pH de la composition est inférieur ou égal à 9,7, la composition étant exempte de persels.

2. Composition selon la revendication 1, **caractérisée en ce que** le peroxyde d'hydrogène représente de 0,1 % à 25 % en poids, de préférence de 1 % à 20 % en poids ou même plus préférentiellement de 2 % à 15 % en poids, par rapport au poids total de la composition.

3. Composition selon l'une quelconque des revendications précédentes, **caractérisée en ce qu'**elle est exempte de générateurs de sel(s) peroxygéné(s).

4. Composition selon l'une quelconque des revendications précédentes, **caractérisée en ce que** le ou les carbonates et/ou le ou les systèmes générateurs de carbonate sont choisis parmi les carbonates de métaux alcalins ($Met_2^+$, $CO_3^{2-}$), de métaux alcalino-terreux (Met' $^{2+}$, $CO_3^{2-}$) ou de phosphonium ($(R''_4P^+)_2$, $CO_3^{2-}$, Met' représentant un métal alcalino-terreux et Met représentant un métal alcalin, et R'', qui peuvent être identiques ou différents, représentant un atome d'hydrogène ou un groupe ($C_1$-$C_6$)alkyle éventuellement substitué tel qu'hydroxyéthyle ; et leurs mélanges.

5. Composition selon l'une quelconque des revendications précédentes, **caractérisée en ce que** le ou les carbonates et/ou le ou les systèmes de génération de carbonate sont choisis parmi les carbonates de métaux alcalins ou de métaux alcalino-terreux et leurs mélanges ; préférentiellement les carbonates de métaux alcalins, et leurs mélanges.

6. Composition selon l'une quelconque des revendications précédentes, **caractérisée en ce que** le ou les carbonates et/ou le ou les systèmes de génération de carbonate sont choisis parmi les carbonates de Na, K, Mg, Ca et leurs mélanges, de préférence le bicarbonate de sodium et/ou de potassium.

7. Composition selon l'une quelconque des revendications précédentes, **caractérisée en ce que** le ou les carbonates représentent de 0,01 % à 20 % en poids, par rapport au poids total de la composition pour l'éclaircissement, et même plus préférentiellement de 0,1 % à 15 % et mieux de 0,5 % à 10 % en poids, par rapport au poids total de la composition.

8. Composition selon l'une quelconque des revendications précédentes, **caractérisée en ce que** ledit ou lesdits dérivés polyphosphorés sont choisis parmi les composés linéaires ou cycliques comprenant au moins deux atomes de phosphore liés entre eux de manière covalente par l'intermédiaire d'au moins un lieur L comprenant au moins un atome d'oxygène et/ou au moins un atome de carbone, de préférence au moins un atome d'oxygène.

9. Composition selon l'une quelconque des revendications précédentes, **caractérisée en ce que** ledit ou lesdits dérivés polyphosphorés sont choisis parmi les composés linéaires ou cycliques comprenant au moins deux atomes de phosphore liés entre eux de manière covalente par l'intermédiaire d'au moins un lieur L comprenant au moins un atome d'oxygène.

**10.** Composition selon l'une quelconque des revendications précédentes, **caractérisée en ce que** ledit ou lesdits dérivés polyphosphorés comprennent au moins deux groupes choisis parmi un groupe -P(R) (=O)-OH, un groupe -P(R) (=O)-O M, un groupe >P(=O)-OH et/ou un groupe >P(=O)-O M,

- M représentant un contre-ion cationique, de préférence choisi parmi les métaux alcalins et les métaux alcalino-terreux,
- R représentant un groupe hydroxyle, un groupe -$O^-$ M, M représentant un contre-ion cationique, de préférence choisi parmi les métaux alcalins et les métaux alcalino-terreux, un groupe ($C_1$-$C_6$) alkyle, ($C_1$-$C_6$) alcoxy, cycloalkyloxy ou (hétéro)aryloxy, et
- > représentant les deux liaisons reliées à l'atome de phosphore et formant partie d'un cycle.

**11.** Composition selon l'une quelconque des revendications précédentes, **caractérisée en ce que** ledit ou lesdits dérivés polyphosphorés sont choisis parmi les composés appartenant à l'une quelconque des formules (I), (II) et (III) ci-dessous ; ou leurs mélanges :

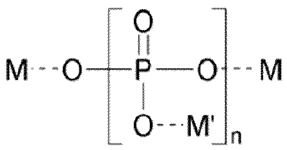

**(I)**

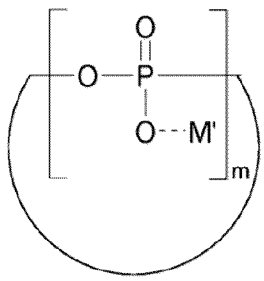

**(II)**

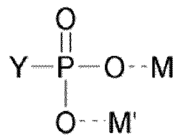

**(III)**

et également leurs solvates tels que les hydrates ;
avec

- n allant de 2 à 10, de préférence de 2 à 6 et mieux de 2 à 3 ;
- m allant de 2 à 10, de préférence de 2 à 6 ;
Y représentant une chaîne alkyle comprenant au moins un atome de phosphore et éventuellement un ou plusieurs hétéroatomes non phosphorés, ou un radical cyclique carboné comprenant éventuellement un ou plusieurs hétéroatomes, ledit radical hydrocarboné étant substitué par un ou plusieurs groupes comprenant un ou plusieurs atomes de phosphore ;
- M ou M' représentant un atome d'hydrogène, un métal alcalin ou un métal alcalino-terreux ;
- - - - - représentant une simple liaison lorsque M ou M' est H, ou une liaison ionique.

**12.** Composition selon l'une quelconque des revendications précédentes, **caractérisée en ce que** le ou les dérivés polyphosphorés sont choisis parmi :

- dérivés polyphosphorés inorganiques choisis parmi :

  o pyrophosphates, de préférence sous forme de sels, de préférence de sels de métaux alcalins, qui peuvent être hydratés ou non, tels que le pyrophosphate de sodium, le pyrophosphate de potassium ou le pyrophosphate de sodium décahydraté ;
  o hexamétaphosphates, de préférence sous forme de sels, de préférence de sels de métaux alcalins, qui peuvent être hydratés ou non, tels que l'hexamétaphosphate de sodium ;
  o tripolyphosphates, de préférence sous forme de sels, de préférence de sels de métaux alcalins, qui peuvent être hydratés ou non, tels que le tripolyphosphate de sodium ;
  o trimétaphosphates, de préférence sous forme de sels, de préférence de sels de métaux alcalins, qui peuvent être hydratés ou non, tels que le trimétaphosphate de sodium ;

- et leurs mélanges ;
- et les dérivés polyphosphorés organiques, choisis de préférence parmi :

  o dérivés de polyphosphates organiques, tels que les acides polyphosphoriques et/ou leurs sels tels que

l'acide phytique ;
o dérivés de polyphosphonate organique, tels que les acides polyphosphoniques et/ou leurs sels tels que EDTMP, DETMP, ATMP, HEDP, DTPMP, et leurs mélanges ;

- et leurs mélanges.

13. Composition selon l'une quelconque des revendications précédentes, **caractérisée en ce que** le ou les dérivés polyphosphorés sont choisis parmi :

- les dérivés de polyphosphate inorganique choisis parmi les pyrophosphates de métaux alcalins hydratés ou non hydratés, tels que le pyrophosphate de sodium, le pyrophosphate de potassium, le pyrophosphate de sodium décahydraté ; et les polyphosphates tels que l'hexamétaphosphate de sodium, le polyphosphate de sodium, le tripolyphosphate de sodium, le trimétaphosphate de sodium ; et leurs mélanges ;
- les dérivés polyphosphorés organiques choisis parmi les acides polyphosphoriques et/ou leurs sels tels que l'acide phytique, les acides polyphosphoniques et/ou leurs sels tels que EDTMP, DETMP, ATMP, HEDP, DTPMP, et leurs mélanges ;
- et leurs mélanges ;

de préférence parmi des dérivés de polyphosphate inorganique.

14. Composition selon l'une quelconque des revendications précédentes, **caractérisée en ce que** le ou les dérivés polyphosphorés sont choisis parmi les dérivés de polyphosphate inorganique, de préférence parmi les pyrophosphates de métaux alcalins hydratés ou non hydratés, de préférence parmi le pyrophosphate de sodium, le pyrophosphate de potassium, le pyrophosphate de sodium décahydraté, et leurs mélanges.

15. Composition selon l'une quelconque des revendications précédentes, **caractérisée en ce que** le ou les dérivés polyphosphorés représentent de 0,5 % à 20 % en poids, par rapport au poids de la composition pour l'éclaircissement les contenant, de préférence de 0,55 % à 15 % en poids, mieux de 0,7 % à 12 % en poids.

16. Composition selon l'une quelconque des revendications précédentes, **caractérisée en ce que** le ou les dérivés polyphosphorés sont présents en une teneur totale allant de 1 % à 10 % en poids, par rapport au poids total de la composition.

17. Composition selon l'une quelconque des revendications précédentes, **caractérisée en ce que** le ou les dérivés polyphosphorés sont présents en une teneur totale de 2 % à 10 % en poids, de préférence de 2,5 % à 10 % en poids par rapport au poids total de la composition.

18. Composition selon l'une quelconque des revendications précédentes, **caractérisée en ce qu'**elle comprend un ou plusieurs tensioactifs, de préférence choisis parmi les tensioactifs anioniques et/ou non ioniques, de préférence des tensioactifs non ioniques.

19. Composition selon l'une quelconque des revendications précédentes, **caractérisée en ce qu'**elle comprend une ou plusieurs substances grasses, de préférence des substances grasses non de silicone, de préférence choisies parmi les hydrocarbures ayant plus de 16 atomes de carbone, les alcanes en $C_6$-$C_{16}$, les huiles ou triglycérides d'origine végétale, les triglycérides liquides de synthèse, les alcools gras, les esters d'acide gras et/ou d'alcool gras autres que les triglycérides, et les cires non de silicone, ou leurs mélanges, de préférence choisis parmi les hydrocarbures à plus de 16 atomes de carbone, tels que l'huile de vaseline, l'huile de paraffine et les polydécènes ; les alcools gras liquides tels que l'octyldodécanol, les acides gras, les esters liquides d'acides gras et/ou d'alcools gras, ou leurs mélanges.

20. Composition selon la revendication précédente, **caractérisée en ce que** la ou les substances grasses représentent une teneur totale allant de 5 % à 80 % en poids, plus préférentiellement de 10 % à 80 % en poids, de préférence de 15 % à 75 % en poids, mieux de 20 % à 70 % en poids, encore plus avantageusement de 25 % à 70 % et préférentiellement de 25 % à 60 % en poids par rapport au poids total de la composition.

21. Composition selon l'une quelconque des revendications précédentes, **caractérisée en ce qu'**elle comprend un ou plusieurs solvants organiques liquides, de préférence ayant une valeur du paramètre de solubilité de Hansen $\delta H$ supérieure à 0 et inférieure à 16 Mpa1/2, de préférence choisis parmi les éthers d'alcool, les esters aliphatiques, les éthers aliphatiques, les éthers aromatiques, les alcanols portant des substituants aryle, les lactones, et leurs

mélanges, de préférence choisis parmi les dérivés du propylèneglycol et les alcools aromatiques, et leurs mélanges, de préférence l'alcool benzylique et/ou l'éther n-butylique du propylèneglycol, de préférence en une teneur totale allant de 0,1 % à 35 % en poids, de préférence de 0,1 % à 20 % en poids et mieux de 0,5 % à 10 % en poids par rapport au poids total de la composition.

**22.** Composition selon l'une quelconque des revendications précédentes, **caractérisée en ce que** le pH est dans la plage de 7 à 9,7, de préférence le pH se situe dans la plage entre 7 et 8,7.

**23.** Composition selon l'une quelconque des revendications précédentes, **caractérisée en ce qu'**elle comprend au moins un agent basifiant d), de préférence choisi parmi i) les alcanolamines telles que la monoéthanolamine, la diéthanolamine, la triéthanolamine et leurs dérivés, ii) les éthylènediamines oxyéthylénées et/ou oxypropylénées, iii) les hydroxydes minéraux ou organiques, iv) les silicates de métaux alcalins tels que les métasilicates de sodium, v) les acides aminés ; et leurs mélanges.

**24.** Procédé pour l'éclaircissement de matières kératiniques, en particulier de fibres kératiniques, de préférence de fibres kératiniques humaines telles que les cheveux, dans lequel la composition telle que définie selon l'une quelconque des revendications 1 à 23 est appliquée sur lesdites matières kératiniques, de préférence les fibres kératiniques.

**25.** Procédé d'éclaircissement selon la revendication précédente, **caractérisé en ce que** la composition telle que définie selon l'une quelconque des revendications 1 à 23 dérive du mélange d'au moins deux compositions, et en particulier de deux compositions :

- une composition (A) comprenant (a) du peroxyde d'hydrogène, et
- une composition (B) comprenant un ou plusieurs carbonates et/ou un ou plusieurs systèmes de génération de carbonate, et (c) un ou plusieurs dérivés polyphosphorés tels que définis selon l'une quelconque des revendications 1 à 23,

de sorte que la teneur en c) dérivé(s) polyphosphoré(s) de la composition résultant du mélange des compositions (A) + (B) soit supérieure ou égale à 0,5 % en poids par rapport au poids total de la composition, et de sorte que le pH de la composition résultant du mélange des compositions (A) + (B) soit inférieur ou égal à 9,7, de préférence inférieur ou égal à 9,5, et de sorte que la composition résultant du mélange des compositions (A) + (B) soit exempte de persels.

**26.** Dispositif à plusieurs compartiments comprenant au moins un premier compartiment contenant la composition (A) telle que définie dans la revendication 25 et au moins un second compartiment contenant la composition (B) telle que définie dans la revendication 25, les compositions des compartiments étant destinées à être mélangées avant application, pour donner la formulation après mélange, à condition que la teneur en c) dérivé(s) polyphosphoré(s), tels que définis selon l'une quelconque des revendications 1 à 22, de la composition résultant du mélange des compositions (A) + (B) soit supérieure ou égale à 0,5 % en poids par rapport au poids de la formulation issue du mélange de (A) + (B) et que le pH de la formulation résultant du mélange de (A) + (B) soit inférieur ou égal à 9,7, de préférence inférieur ou égal à 9,5, et que la composition résultant du mélange des compositions (A) + (B) soit exempte de persels.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- FR 2961397
- US 4874554 A
- US 4137180 A
- WO 9515144 A
- WO 9501772 A
- EP 714954 A


### Non-patent literature cited in the description


- Walter Noll's Chemistry and Technology of Silicones. Academic Press, 1968
- **TODD** ; **BYERS**. Volatile Silicone Fluids for Cosmetics. *Cosmetics and Toiletries*, vol. 91, 27-32
- **M.R. PORTER**. Handbook of Surfactants. Blackie & Son, 1991, 116-178
- **CHARLES M. HANSEN**. Hansen solubility parameters: A User's Handbook. CRC Press, 2000, 167-185
- Handbook of Solubility Parameters and Other Cohesion Parameters. CRC Press, 95-121, 177-185
- **BAGDA, E**. The relation between surface tension and solubility parameter in liquids. *Farbe Lack*, 1978, vol. 84, 212